# EUROPEAN PATENT APPLICATION

(11) **EP 4 796 128 A1**
(43) Date of publication of application: **26.08.2026**
(21) Application number: 24880188.8
(22) Date of filing: 18.10.2024
(51) Int. Cl.: A61K 47/68, A61P 35/00, C07K 16/28

(54) **ANTI-C-MET ANTIBODY-DRUG CONJUGATE COMPRISING CAMPTOTHECIN DERIVATIVE**

(30) Priority: 19.10.2023 KR 20230140521
(71) Applicant: Celltrion, Inc., Incheon 22014 (KR)
(72) Inventor: YANG, Sungjae, Incheon 22014 (KR); KIM, Woogyum, Incheon 22014 (KR); CHO, Jinhwan, Incheon 22014 (KR); YOO, Byounggyu, Incheon 22014 (KR); HWANG, Boram, Incheon 22014 (KR); YOON, Joonsun, Incheon 22014 (KR); JUNG, Doo Young, Suwon-si, Gyeonggi-do 16506 (KR); CHO, Hyun Yong, Suwon-si, Gyeonggi-do 16506 (KR); LEE, Byeong Sung, Suwon-si, Gyeonggi-do 16506 (KR); CHUN, Young Hwa, Suwon-si, Gyeonggi-do 16506 (KR); SEO, Jimin, Incheon 22014 (KR); KIM, Jongin, Incheon 22014 (KR); JUNG, Myungho, Incheon 22014 (KR)
(74) Representative: Maiwald GmbH
(86) International application number: PCT/KR2024/015890
(87) International publication number: WO 2025/084851

(57) **Abstract**

The present invention provides an antibody-drug conjugate having a structure in which a compound according to chemical formula 1 or a pharmaceutically acceptable salt thereof, stereoisomer, tautomer, prodrug, hydrate, solvate, or isotope-labeled analog thereof, is linked to an anti-c-Met antibody via a linker. The antibody-drug conjugate according to the present invention can selectively exhibit the effect of the compound of chemical formula 1 on cancer cells expressing c-Met.

## Description

### Technical Field

The present invention relates to an anti-c-Met antibody-drug conjugate having a structure in which a compound according to Formula 1 is linked to an antibody via a linker.

In addition, the present invention relates to a method for treating or preventing cancer using the antibody-drug conjugate, and relates to a pharmaceutical composition for treating or preventing cancer comprising the antibody-drug conjugate as an active ingredient, a method for treating or preventing cancer comprising administering the same, and a use thereof.

### Background Art

Cancer is one of the most serious diseases, and according to the statistics from the World Health Organization (WHO), cancer incidence is increasing every year worldwide, thus requiring the development of new anticancer drugs in the pharmaceutical industry. In this regard, camptothecin (CPT), which is an alkaloid isolated from a plant of the family *Davidiaceae*, has been identified to have efficacy as a cytotoxic anticancer substance by inhibiting topoisomerase, and camptothecin drugs, such as irinotecan (CPT-11), topotecan, 10-hydroxy camptothecin, belotecan (CKD-602), and exatecan have been developed domestically and internationally. In addition, a substance called DXd has been recently developed, and the development of new camptothecin derivatives is continuously progressing.

However, since cytotoxic anticancer agents lack cell selectivity, they attack normal cells as well as cancer cells, thereby causing unexpected side effects. Therefore, cytotoxic anticancer agents are often prepared as antibody-drug conjugates (ADCs) by conjugating antibodies that bind to specific target antigens on the surface of cancer cells with drugs that have cytotoxic effects.

Meanwhile, c-MET protein is a protein belonging to the Met family and is a type of tyrosine kinase receptor expressed on the surface of various epithelial cells. Hepatocyte growth factor (HGF) is a ligand for the c-MET receptor, and the binding of HGF to c-MET initiates a series of intracellular signals that mediate embryonic development and wound healing in normal cells. However, in various types of major cancer cells, comprising breast cancer, gastric cancer, lung cancer, and colon cancer, the amplification, mutation, and protein overexpression related to the c-Met gene occur, thus promoting tumor development and progression. Therefore, c-Met and its related signaling pathways are attracting attention as clinically important therapeutic targets in anticancer treatment.

Accordingly, the development of antibody-drug conjugates that have high cytotoxicity and specifically act on cancer cells expressing c-Met, by comprising camptothecin derivative compounds as drugs, is becoming a new challenge.

### Disclosure of Invention

### Technical Problem

Therefore, an object of the present invention is to provide an anti-c-Met antibody-drug conjugate, comprising a camptothecin derivative having excellent cytotoxicity activity and excellent target specificity for cells expressing c-Met, and pharmaceutical uses thereof.

### Solution to Problem

In order to solve the above problems, the present invention provides an antibody-drug conjugate (ADC) having a structure, in which a compound according to Formula 1 or a pharmaceutically acceptable salt, a stereoisomer, a tautomer, a prodrug, a hydrate, a solvate, or an isotopically labeled analog thereof is linked to an anti-c-Met antibody via a linker.

Another aspect of the present invention provides a method for treating or preventing cancer, comprising contacting the antibody-drug conjugate with cancer cells.

A still another aspect of the present invention provides a pharmaceutical composition for treating or preventing cancer, comprising the antibody-drug conjugate as an active ingredient; a method for treating or preventing cancer comprising administering the same; and a use thereof.

### Advantageous Effects of Invention

The antibody-drug conjugate according to the present invention can exhibit the effect of a compound drug of Formula 1 in a target-specific manner on cells expressing c-Met.

The compound of Formula 1, which is an active camptothecin derivative designed to bind to DDX5 protein and E3 ligase, may be one that is able to bind to E3 ligase as a molecular glue; and/or is able to kill target cells expressing DDX5 protein via a molecular glue mechanism of action (MoA); and/or has a mechanism of action (MoA) that degrades oncoprotein DDX5 along with the ability to inhibit type 1 topoisomerase.

The antibody-drug conjugate of the present invention can easily penetrate cell membranes due to the hydrophobicity of the compound of Formula 1, and when it enters cancer cells, the linker is specifically cleaved by a lysosomal protease highly expressed in tumors, thereby releasing the drug of Formula 1 and killing the cancer cells. The drug can then be released and cause a bystander effect that kills surrounding cancer cells. This allows the drug to exhibit an excellent killing effect not only on cancer cells that highly express c-Met, but also on cancer cells that have low expression.

### Brief Description of Drawings

Fig. 1 shows the results of size exclusion chromatography (SEC) of the antibody-drug conjugate (ADC) of Example 3.
Fig. 2 shows the results of flow cytometry analysis for commercially available monoclonal antibodies (FITC-mAb, 11-8858-42), anti-c-Met antibody, and FITC-anti human IgG Fc (410720) antibody in the process of selecting a cell line expressing c-Met protein.
Fig. 3 shows the results of comparative evaluation of *in-vitro* cell viability when a cytotoxic compound or ADC was treated in T47D (a breast cancer cell line).
Fig. 4 shows the results of comparative evaluation of *in-vitro* cell viability when a cytotoxic compound or ADC was treated in SNU-5 (a gastric cancer cell line).
Fig. 5 shows the results of comparative evaluation of *in-vitro* cell viability when a cytotoxic compound or ADC was treated in NCI-H1573 (a lung cancer cell line).
Fig. 6 shows the results of comparative evaluation of *in-vitro* cell viability when a cytotoxic compound or ADC was treated in HT29 (a colon cancer cell line).
Fig. 7 shows the results of comparative evaluation of *in-vitro* cell viability when a cytotoxic compound or ADC was treated in NCI-H1975 (a lung cancer cell line).
Fig. 8 shows a graph illustrating the binding affinity of the M01 antibody to c-Met proteins derived from humans, mice, rats, dogs, and monkeys.
Fig. 9 shows graphs illustrating the binding affinity of ADC (M01-PBX-7016) of Example 3 and the M01 antibody to c-Met proteins derived from humans and monkeys.
Fig. 10 shows a graph illustrating that the M01 antibody binds to cell lines expressing c-Met protein in a dose-dependent manner.
Fig. 11 shows a graph illustrating the change in body weight of mice during the period of *in-vivo* experiment on Hs746T (a gastric cancer cell line).
Figs. 12 and 13 each show a graph illustrating tumor growth curves during the period of *in-vivo* experiment on Hs746T (a gastric cancer cell line).
Fig. 14 shows a graph illustrating the change in body weight of mice during the period of *in-vivo* experiment on LU-01-0439 (a lung cancer cell line).
Fig. 15 shows a graph illustrating tumor growth curves during the period of *in-vivo* experiment on LU-01-0439 (a lung cancer cell line).
Fig. 16 shows a graph illustrating the change in body weight of mice during the period of *in-vivo* experiment on NCI-H441 (a lung cancer cell line).
Fig. 17 shows a graph illustrating tumor growth curves during the period of *in-vivo* experiment on NCI-H441 (a lung cancer cell line).

### Best Mode for Carrying out the Invention

The present invention will be described in more detail below.

The embodiments of the present invention may be modified in various different forms, and the scope of the present invention is not limited to the embodiments described below. Additionally, the embodiments of the present invention are provided to more completely explain the present invention to a person of ordinary knowledge in the art. Furthermore, to "comprise" a component throughout a specification means that, unless otherwise specifically stated, it may include other elements, rather than excluding other elements.

According to an aspect of the present invention, there is provided an antibody-drug conjugate (ADC) having a structure, in which a compound according to Formula 1 below or a pharmaceutically acceptable salt, a stereoisomer, a tautomer, a prodrug, a hydrate, a solvate, or an isotopically labeled analog thereof is linked to an anti-c-Met antibody via a linker: 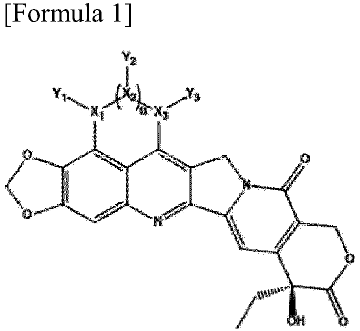 wherein:
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
n may be 0, 1, or 2; and
Y₁, Y₂, and Y₃ may each independently be hydrogen or a functional group comprising oxygen, nitrogen, phosphorus, or sulfur.

In this case, when X₂ is a single bond or double bond, n may be 0. More specifically, it may be a single bond or double bond linking X₁ and X₃.

In particular, non-limiting examples of the functional group comprising oxygen, nitrogen, phosphorus, or sulfur may be those which comprise a functional group selected from the group consisting of -CHO, -COOH, -NH₂, -SH, -CONH₂, -PO₃H, - PO₄H₂, -OPO₄H, -PO₂(OR¹)(OR²) (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -SO₃H, -OSO₃H, - NO₂, -N₃, -NR₃OH(R=CₙH₂ₙ₊₁, 0≤n≤16), -NR₃⁺X⁻(R= CₙHₘ, 0≤n≤16, 0≤m≤ 34, X = OH, Cl, or Br), NR₄⁺X⁻(R= CₙHₘ, 0≤n≤16, 0≤m≤34, X = OH, Cl, or Br), -COSH, - COOCO-, -CORCO- (R = CₙHₘ, 0≤n≤3, 0≤m≤2l+1), -COOR, -CN, -N₃, -N₂, - NROH(R = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹NR²R³ (R¹, R², R³ = CₛHₜNᵤO_{w}SₓP_{y}X_{z}, X = - F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), - CONHNR¹R² (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹R²R³X' (R¹, R², R³ = CₛHₜNᵤO_{w}SₓP_{y}X_{z}, X = -F, -Cl, -Br, or -I, X' = F-, Cl-, Br-, or I-, 0≤s≤20, 0≤t≤2(s+u)+1, 0≤u≤2s, 0≤w≤2s, 0≤x≤2s, 0≤y≤2s, 0≤z≤2s), -OH, -O-, >C=O, -SS-, -SO-, -NO₂, -COX (X = F, Cl, Br, or I), -COOCO-, -CONH-, -CN, -SCOCH₃, -SCN, -NCS, -NCO, -OCN, -CN, -F, -Cl, -I, -Br, an epoxy group, -hydrazone, -ONO₂, -PO(OH)₂, -C=NNH₂, - HC=CH-, -C=C-, -C≡C-, and a hydrocarbon with two or more carbon atoms.

According to an aspect of the present invention, there is provided an antibody-drug conjugate (ADC) having a structure, in which a compound according to Formula 1 below or a pharmaceutically acceptable salt, a stereoisomer, a tautomer, a prodrug, a hydrate, a solvate, or an isotopically labeled analog thereof is linked to an anti-c-Met antibody via a linker:

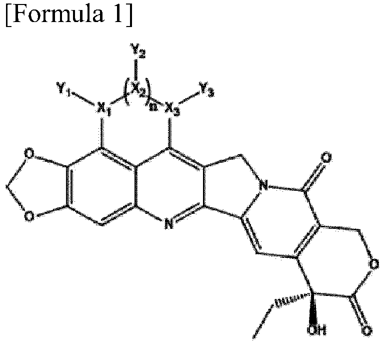

In particular,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
X₁, (X₂)ₙ, and X₃ may together form a 6-membered or 7-membered ring, where n is 1 or 2; and
Y₁, Y₂, and Y₃ may each independently be hydrogen or a functional group comprising oxygen, nitrogen, phosphorus, or sulfur.

In particular, non-limiting examples of the functional group comprising oxygen, nitrogen, phosphorus, or sulfur may be those which comprise -CHO, -COOH, -NH₂, - SH, -CONH₂, -PO₃H, -PO₄H₂, -OPO₄H, -PO₂(OR¹)(OR²)(R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -SO₃H, -OSO₃H, -NO₂, -N₃, -NR₃OH (R = CₙH₂ₙ₊₁, 0≤n≤16), -NR₃⁺X⁻ (R= CₙHₘ, 0≤n≤16, 0≤m≤ 34, X = OH, Cl, or Br), NR₄⁺X⁻(R= CₙHₘ, 0≤n≤16, 0≤m≤34, X = OH, Cl, or Br), -COSH, -COOCO-, -CORCO- (R = CₙHₘ, 0≤n≤3, 0≤m≤2l+1), -COOR, - CN, -N₃, -N₂, -NROH(R = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹NR²R³(R¹, R², R³ = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -CONHNR¹R² (R¹, R² = CₛHₜNᵤO_{w}SₓP_{y}X_{z}, X = -F, -Cl, -Br, or -I, 0≤s≤20, 0≤t≤2(s+u)+1, 0≤u≤2s, 0≤w≤2s, 0≤x≤2s, 0≤y≤2s, 0≤z≤2s), -NR¹R²R³X' (R¹, R², R³ = CₛHₜNᵤO_{w}SₓP_{y}X_{z}, X = -F, -Cl, -Br, or -I, X'= F-, Cl-, Br, or I-, 0≤s≤20, 0≤t≤2(s+u)+1, 0≤u≤2s, 0≤w≤2s, 0≤x≤2s, 0≤y≤2s, 0≤z≤2s), -OH, -O-, >C=O, -SS-, - SO-, -NO₂, -COX (X = F, Cl, Br, or I), -COOCO-, -CONH-, -CN, -SCOCH₃, -SCN, - NCS, -NCO, -OCN, -CN, -F, -Cl, -I, -Br, an epoxy group, -hydrazone, -ONO₂, - PO(OH)₂, -C=NNH₂, -HC=CH-, -C=C-, -C≡C-, and a hydrocarbon with two or more carbon atoms.

According to an aspect of the present invention, in Formula 1 above,
X₁, X₂, and X₃ may each independently be carbon;
n may be 1;
Y₁ and Y₂ may each independently be hydrogen; and
Y₃ may be hydrogen, or a functional group comprising oxygen, nitrogen, phosphorus, or sulfur.

In this case, non-limiting examples of the functional group comprising oxygen, nitrogen, phosphorus, or sulfur may be the same as the examples above.

According to an aspect of the present invention, in Formula 1 above,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
n may be 0, 1, or 2; and
Y₁, Y₂, and Y₃ may each independently be hydrogen or a functional group comprising oxygen, nitrogen, phosphorus, or sulfur.

The functional group comprising nitrogen may be -NZ₁Z₂; and
Z₁ and Z₂ may each independently be one which comprises a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be hydrogen, or a functional group comprising oxygen, nitrogen, phosphorus, or sulfur.

In this case, non-limiting examples of the functional group comprising oxygen, nitrogen, phosphorus, or sulfur may be the same as the examples above.

According to an aspect of the present invention, in Formula 1 above,
X₁ and X₃ may each independently be carbon, oxygen, nitrogen, or sulfur, and X₁ and X₃ may be the same or different;
X₂ may be carbon, oxygen, nitrogen, sulfur, a single bond, or a double bond;
X₁, (X₂)ₙ, and X₃ may together form a 6-membered or 7-membered ring, where n is 1 or 2; and
Y₁, Y₂, and Y₃ may each independently be hydrogen or a functional group comprising oxygen, nitrogen, phosphorus, or sulfur.

The functional group comprising nitrogen may be -NZ₁Z₂; and
Z₁ and Z₂ may each independently be one which comprises a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ and Z₂ may each independently be one which comprises a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ and Z₂ may each independently be hydrogen, C(=O)H, C(=O)(C₁₋₃ alkyl), C(=O)(C₂₋₃ alkenyl), C(=O)NH₂, C(=O)NH(C₁₋₃ alkyl), orC(=O)CR¹R²R³.

In this case, R¹, R², and R³ may each independently be hydrogen, hydroxyl, C₁₋₃ alkyl, C₃₋₇ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ may be hydrogen, and Z₂ may be C(=O)(C₂₋₃ alkenyl) or C(=O)CR¹R²R³.

In this case, R¹, R², and R³ may each independently be hydrogen, hydroxyl, C₁₋₃ alkyl, C₃₋₅ cycloalkyl, or C₁₋₃ haloalkyl.

According to an aspect of the present invention, in Formula 1 above,
X₁, X₂, and X₃ may each independently be carbon;
n may be 1;
Y₁ and Y₂ may each independently be hydrogen; and
Y₃ may be -NZ₁Z₂; and
Z₁ may be hydrogen, and Z₂ may be C(=O)CR¹R²R³.

In particular, R¹, R², and R³ may each independently be hydrogen, hydroxyl, or C₁₋₃ alkyl.

According to an aspect of the present invention, the compound of Formula 1 above may be a compound represented by any one of Formulas 2 to 11 below, or a pharmaceutically acceptable salt, a stereoisomer, a tautomer, a prodrug, a hydrate, a solvate, or an isotopically labeled analog thereof.

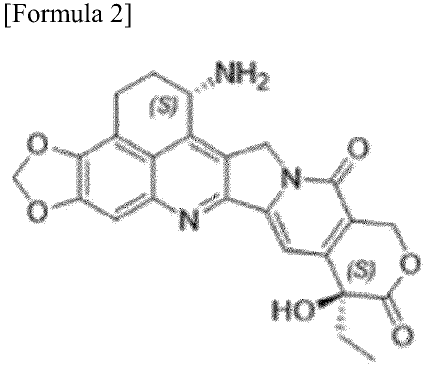

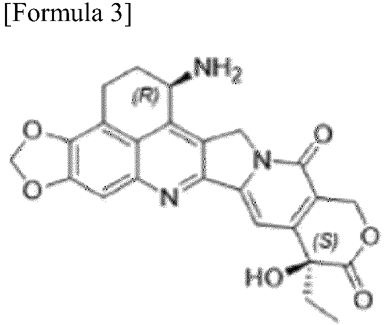

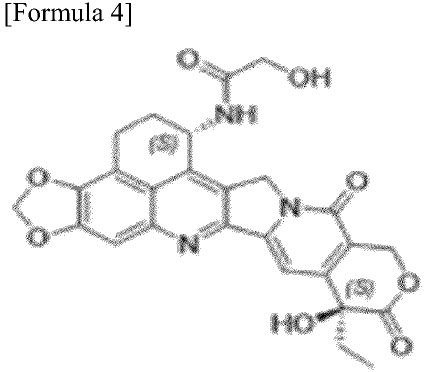

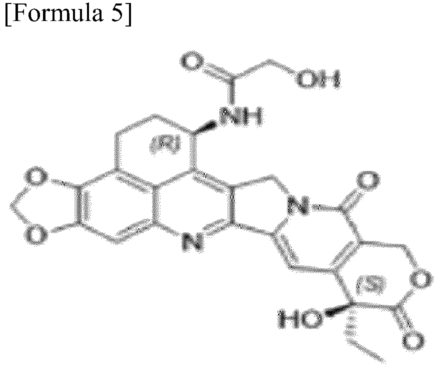

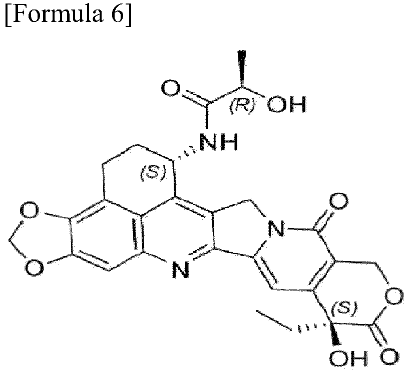

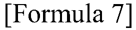

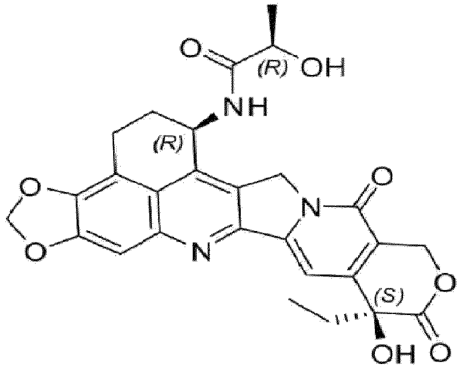

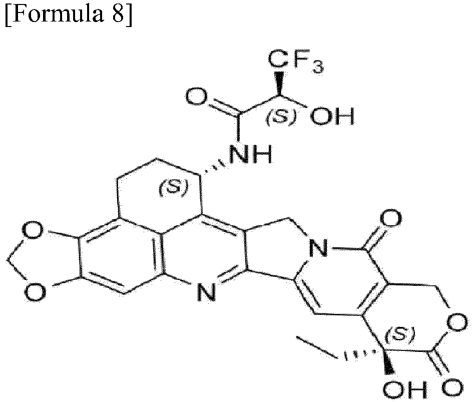

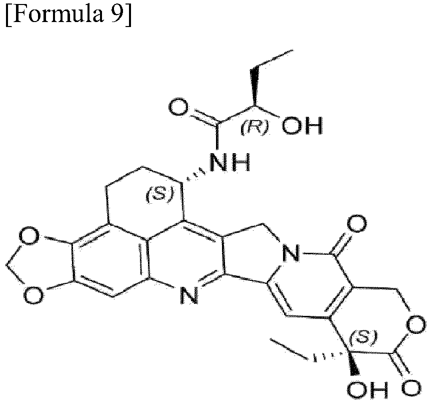

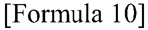

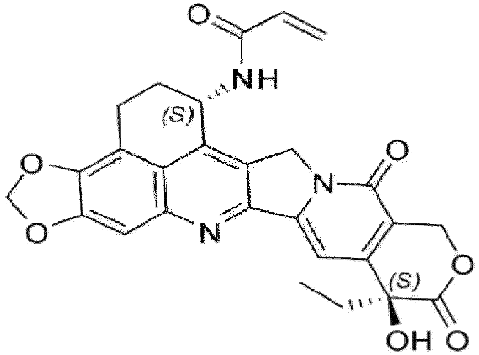

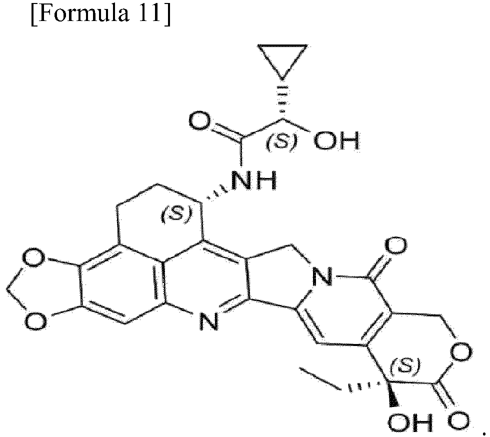

According to an aspect of the present invention, the compound of Formula 1 above may be an active camptothecin derivative designed to bind to DDX5 protein and E3 ligase.

According to an aspect of the present invention, the compound of Formula 1 above may be one which binds to E3 ligase by a molecular glue.

According to an aspect of the present invention, the compound of Formula 1 above may kill the target cells expressing DDX5 protein through a molecular glue mechanism of action (MoA).

The target cells may be cancer cells or senescent cells. Senescent cells also comprise cells that no longer perform the organ's unique characteristic functions.

According to an aspect of the present invention, the compound of Formula 1 above may be one which has a mechanism of action (MoA) that degrades the oncoprotein DDX5 along with the ability to inhibit type 1 topoisomerase.

As used herein, the term "drug" or "payload" may refer to any one compound selected from the group consisting of Formulas 1 to 11. The drug or payload of the present invention may be combined with a linker and an anti-c-Met antibody to form an antibody-drug conjugate.

As used herein, the term "antibody-drug conjugate (ADC)" may enable the linkage of a monoclonal antibody or an antigen-binding fragment thereof with a biologically active cytotoxin via a chemically stable linker, and thereby avoid the low efficacy of antibody therapeutics or the toxicity issue of cytotoxin, while maximizing the specificity of antibody binding to surface antigens of cancer cells and the high efficiency of cytotoxin. This allows ADCs to accurately bind to cancer cells and reduce their impact on normal cells, compared to conventional anticancer treatments. The antibody-drug conjugate of the present invention specifically binds to cells expressing c-Met, and thus can specifically target drugs.

The antibody-drug conjugate is characterized in that it can easily penetrate cell membranes due to the hydrophobicity of the compound of Formula 1, and in that when it enters cancer cells, the linker is specifically cleaved by a lysosomal protease highly expressed in tumors, thereby releasing the drug of Formula 1 and killing the cancer cells.

The antibody-drug conjugate is characterized by having an excellent killing effect even on cancer cells with low c-Met expression, by causing a bystander effect, in which the drug is released after cell death and kills surrounding cancer cells.

As used herein, the term "antibody" may refer to an antibody or an antigen-binding fragment thereof, or may refer to a macromolecule compound capable of recognizing and binding to an antigen or receptor associated with a population of target cells to which it binds. The antibody may be a murine antibody, a humanized antibody, a chimeric antibody, a rabbit antibody, a phage display antibody, a yeast display antibody, a CDR grafted antibody, or a recombinant human antibody, but is not limited thereto. In particular, the term antibody used herein refers to "anti-c-Met antibody", and this may refer to an antibody or an antigen-binding fragment thereof that specifically binds to c-Met.

The antibody of the present invention may preferably be a monoclonal antibody, and the antigen-binding fragment may be one or more fragments of an antibody having specific binding ability to an antigen.

The antibody of the present invention may form a bond with a linker through a heteroatom on the antibody.

The antibody of the present invention is an anti-c-Met antibody, and non-limiting examples of the anti-c-Met antibody may comprise telisotuzumab, emibetuzumab (LY2875358), onartuzumab, ARGX-111, HLX55, and M01. In particular, the M01 may be an antibody having CDRs of SEQ ID NOs: 1 to 6. In an aspect of the present invention, the M01 may be an antibody which comprises i) a heavy chain having an amino acid sequence of SEQ ID NO: 9 and ii) a light chain having an amino acid sequence of SEQ ID NO: 10.

The antibody of the present invention may be an antibody having i) a heavy chain comprising CDR-H1, CDR-H2, and CDR-H3 having the amino acid sequences of SEQ ID NOs: 1, 2, and 3, respectively, and ii) a light chain comprising CDR-L1, CDR-L2, and CDR-L3 having amino acid sequences of SEQ ID NOs: 4, 5, and 6, respectively; or an antigen-binding fragment thereof.

The antibody of the present invention may be an antibody comprising i) a heavy chain variable region having the amino acid sequence of SEQ ID NO: 7 and ii) a light chain variable region having the amino acid sequence of SEQ ID NO: 8; or an antigen-binding fragment thereof.

The antibody of the present invention may be an antibody comprising i) a heavy chain variable region having the amino acid sequence of SEQ ID NO: 9 and ii) a light chain variable region having the amino acid sequence of SEQ ID NO: 10; or an antigen-binding fragment thereof.

The drug to antibody ratio (DAR), which refers to the average number of drugs linked per antibody of the present invention, may be in a range of about 1 to about 20, and specifically, may be in a range of about 1 to about 8, about 2 to about 8, about 3 to about 8, about 4 to about 8, about 5 to about 8, about 6 to about 8, about 7 to about 8, or about 7.5 to about 8. Additionally, the DAR may specifically be in a range of about 3 to about 5, about 3.5 to about 4.5, or about 3 to about 4.

In an aspect of the present invention, the DAR of the antibody-drug conjugate of the present invention may be in a range of 7 to 8 or 3 to 4. In a preferred aspect of the present invention, the DAR of the antibody-drug conjugate of the present invention may be in a range of 7 to 8. In a preferred aspect of the present invention, the DAR of the antibody-drug conjugate of the present invention may be in a range of 3 to 4.

As used herein, the term "linker" refers to a chemical structural fragment or bond which is linked to an antibody at one end and to a drug at the other end, or linked to another linker and then to a drug.

More specifically, the linker may be cleavable or non-cleavable, and the cleavable linker not only functions to link the antibody and the drug together, but may also be selectively cleaved by at specific pH, enzymes (protease, phosphatase, glycosidase, and sulfatase), and a glutathione (GSH) level of the cancer-surrounding environment to thereby release the drug.

More specifically, the linker may comprise at least one selected from the group consisting of C₁₋₆ alkylene, carbonyl, maleimide, succinimide -O-, sugar, sugar acid, β-glucuronide, β-galactoside, phosphodiester, PEG, PSAR (polysarcosine), disulfide, PAB (*p*-aminobenzyloxycarbonyl), triazole, hydrazone, phosphate, diester, thioether, and peptide, but is not limited thereto.

More specifically, the peptide residue may be a peptide residue consisting of one or more amino acids selected from the group consisting of alanine (A), asparagine (N), glutamine (Q), ornithine, proline (P), threonine (T), phenylalanine (F), glycine (G), valine (V), lysine (K), citrulline, serine (S), glutamic acid (E), and aspartic acid (D); more specifically, it may be a peptide residue consisting of one or more amino acids selected from the group consisting of valine, citrulline, phenylalanine and glycine; more specifically, it may be a dipeptide, tripeptide, or tetrapeptide residue; and more specifically, it may be a dipeptide residue of valine-citrulline, valine-alanine, and phenylalanine-glycine, or a tetrapeptide residue of GGFG (glycine-glycine-phenylalanine-glycine). In this case, in the peptide residue, some of the amino acid residues may be optionally substituted.

The "linker" of the present invention may be represented by Formula 12 below: 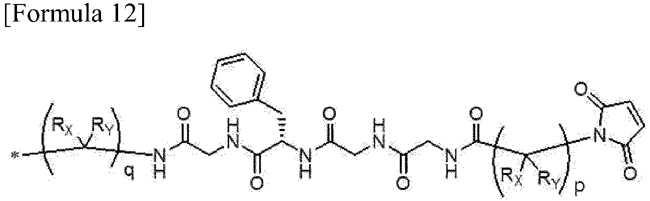 wherein in Formula 12 above,
R_{X} and R_{Y} are each independently hydrogen or C₁₋₆ alkyl;
p and q are each an integer of 1 to 6; and
* is a part that binds to the payload.

According to one aspect of the present invention, in Formula 12 above,
R_{X} and R_{Y} may each independently be hydrogen or C₁₋₃ alkyl;
p may be an integer of 4 to 6; and
q may be an integer of 1 to 3.

According to an aspect of the present invention, in Formula 12 above,
R_{X} and R_{Y} may each independently be hydrogen;
p may be 5; and
q may be 1.

According to an aspect of the present invention, the compound of Formula 12 above may be a compound of Formula 13.

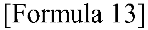

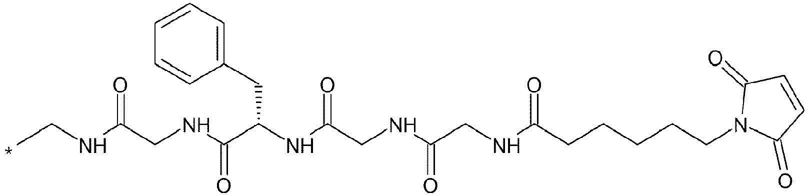

In Formula 13 above, * is a part that binds to the payload.

In an aspect of the present invention, in the compound of Formula 12, the maleimide ring may be linked to an amino acid residue of an antibody. In a preferred aspect of the present invention, in the compound of Formula 12, any one of the carbons of the -CH₂=CH₂- structure of the maleimide ring may bind to cysteine among the amino acid residues of the antibody.

In an aspect of the present invention, when the compound of Formula 13 is combined with an antibody, it may be linked in the form of Formula 14.

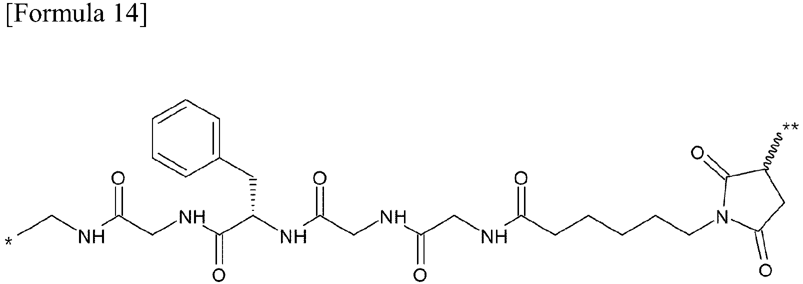

In Formula 14 above, * is a part that binds to the payload, and ** is a part that binds to the antibody.

In an aspect of the present invention, ** indicated in the compound of Formula 14 is a part that binds to an antibody. In a preferred aspect of the present invention, the compound of Formula 14 may bind to cysteine among the amino acid residues of the antibody at the ** part.

As used herein, the term "mc-GGFG" refers to a linker of the structure of a compound of Formula 13 or a compound of Formula 14.

The compound of Formula 1 according to the present invention may exist in the form of a pharmaceutically acceptable salt, stereoisomer, tautomer, prodrug, hydrate, solvate, or isotopically labeled analog. Accordingly, the scope of the compounds of the present invention may comprise the compound of Formula 1 above and a pharmaceutically acceptable salt, a stereoisomer, tautomer, prodrug, hydrate, solvate, or isotope-labeled analogs thereof, and may similarly be applied to drug-linker compounds and antibody-drug conjugates based thereon.

As used herein, the term "pharmaceutically acceptable salt" refers to any organic or inorganic acid addition salt of the compound, in a concentration that provides a relatively non-toxic and harmless to the patient and has an effective action, and the side effects due to the salt do not diminish the beneficial effects of the parent compound.

In particular, the pharmaceutically acceptable salt may be an acid addition salt formed by a free acid. In particular, the acid addition salt may be obtained from inorganic acids (*e.g*., hydrochloric acid, nitric acid, phosphoric acid, sulfuric acid, hydrobromic acid, hydroiodic acid, nitrous acid, phosphorous acid, *etc*.); non-toxic organic acids (*e.g*., aliphatic mono- and dicarboxylates, phenyl-substituted alkanoates, hydroxyalkanoates, and alkanedioates; aromatic acids; aliphatic and aromatic sulfonic acids, *etc*.); and organic acids (*e.g*., trifluoroacetic acid, acetate, benzoic acid, citric acid, lactic acid, maleic acid, gluconic acid, methanesulfonic acid, 4-toluenesulfonic acid, tartaric acid, fumaric acid, *etc*.).

Such pharmaceutically acceptable salts may comprise sulfates, sulfites, nitrates, phosphates, pyrophosphates, chlorides, bromides, iodides, fluorides, acetates, trifluoroacetates, propionates, decanoates, caprylates, acrylates, formates, isobutyrates, caprates, heptanoates, propiolates, oxalates, malonates, succinates, suberates, sebacates, fumarates, maleates, benzoates, phthalates, benzenesulfonates, toluenesulfonates, phenylacetates, phenylpropionates, phenylbutyrates, citrates, lactates, glycolates, malates, tartrates, mandelates, *etc*.

The acid addition salt may be prepared by a conventional method, for example, by dissolving the corresponding compound in an organic solvent (*e.g*., methanol, ethanol, acetone, methylene chloride, acetonitrile, *etc*.), adding an organic acid or inorganic acid thereto, and filtering and drying the resulting precipitate; or by distilling the solvent and an acid in an excess amount under reduced pressure, drying, and crystallizing in the presence of an organic solvent.

Additionally, the pharmaceutically acceptable salt may be a salt obtained using a base or a metal salt. As an example of the metal salt, an alkali metal or alkaline earth metal salt may be obtained by dissolving the compound in a solution with an excess amount of alkali metal hydroxide or alkaline earth metal hydroxide, filtering the undissolved compound salt, and evaporating and drying the filtrate. As the alkali metal salt, sodium, potassium, or calcium salts may be pharmaceutically suitable. Additionally, the corresponding salt may be obtained by reacting to an alkali metal or alkaline earth metal salt with a suitable silver salt (*e.g*., silver nitrate).

Additionally, the compound of the present invention may comprise hydrates and solvates. The hydrate refers to a compound or a salt thereof, which further comprises a stoichiometric or non-stoichiometric amount of water bound by a non-covalent intermolecular force. The hydrate comprises, for example, a monohydrate, a dihydrate, a trihydrate, a tetrahydrate, *etc*. The solvate refers to a solvent-containing compound formed by association of one or more solvent molecules with the compound of the present invention. The solvate comprises, for example, a single solvate, a disolvate, a trisolvate, and a tetrasolvate. The hydrate and the solvate may be prepared using known methods, and are preferably non-toxic and water-soluble. In this case, preferably, the hydrate and solvate may each be water, an alcoholic solvent (especially, methanol, ethanol, *etc*.), trifluoroacetic acid, formic acid, and ammonia, but are not limited thereto.

Additionally, the compound of the present invention may be prepared in the form of a prodrug, which has little or no pharmacological activity itself, but can be converted into a compound having the desired activity, for example, by hydrolysis, when administered into the body. Such prodrugs may be derivatives of compounds comprising a biologically reactive functional group, in which the biologically reactive functional group may be cleaved from the compound or otherwise reacted under biological conditions (*in vivo* or *in vitro*) to provide the compound. Typically, prodrugs are inactive, or at least less active than the compound, which allows the compound to exhibit its activity after cleavage from the biologically reactive functional group. The biologically reactive functional group may be hydrolyzed or oxidized under biological conditions to produce a compound. For example, prodrugs may comprise a biologically hydrolyzable group. Examples of the biologically hydrolyzable groups may comprise biologically hydrolyzable phosphates, biologically hydrolyzable esters, biologically hydrolyzable amides, biologically hydrolyzable carbonic esters, biologically hydrolyzable carbamates, and biologically hydrolyzable ureides, but are not limited thereto.

Additionally, the compound of the present invention may have the form of a stereoisomer. The stereoisomer refers to compounds which have the same formula or molecular formula but are sterically different, and comprise enantiomers, diastereomers, geometric isomers (or *cis*/*trans* isomers), rotamers, and atropisomers, and each of these isomers and mixtures thereof are also comprised in the scope of the present invention. For example, the compound of the present invention may have a chiral carbon center, and thus individual enanatiomers or mixtures thereof (*e.g*., racemic mixtures) may also fall within the scope of the present invention. Additionally, the compound of the present invention may have the form of a conformational isomer (*e.g*., a rotamer) and a diastereomer comprising a geometric isomer (or *cis*/*trans* isomer), and all such stereoisomers and mixtures thereof may all fall within the scope of the present invention. In this aspect, the Formula (I) of the present invention may comprise the stereoisomers of Formula (I) because the stereochemical structure is not specified. Specifically, the solid bond ( 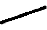 ) being linked to an asymmetric carbon atom in the chemical structural formula may comprise a wedge-shaped solid bond ( 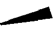 ) and a wedge-shaped dotted bond ( 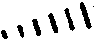 ), which represent the absolute arrangement of the stereogenic center. Additionally, it should be understood that stereoisomers of the exemplary compounds described herein are comprised within the scope of the present invention.

Additionally, the compound of the present invention may exist in the form of a tautomer. Tautomers may have structures in which a proton has shifted from one atom of a molecule to another atom of the same molecule, and may be interconverted by the movement of hydrogen atoms accompanying the conversion of single bonds and adjacent double bonds. In a solution where tautomerization is possible, a chemical equilibrium of tautomers will exist. The exact ratio of tautomers depends on several factors, comprising temperature, solvents, and pH.

Additionally, the compound of the present invention further comprises an isotopically labeled analog. Specifically, the compound of the present invention may comprise isotopes of hydrogen, carbon, nitrogen, oxygen, phosphorus, sulfur, fluorine, and chlorine (²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively). All compounds of the present invention comprising the above-described isotopes and/or other isotopes of other atoms fall within the scope of the present invention.

According to an aspect of the present invention, there is provided a method for treating or preventing cancer, which comprises contacting the antibody-drug conjugate with cancer cells.

The antibody-drug conjugate according to the present invention can inhibit the activity of cancer cells. Therefore, the present invention provides a pharmaceutical composition or anticancer agent for treating or preventing cancer, which comprises the antibody-drug conjugate as an active ingredient.

According to an aspect of the present invention, the present invention provides a method for treating or preventing cancer, comprising administering an antibody-drug conjugate to a subject.

According to an aspect of the present invention, the present invention provides a use of an antibody-drug conjugate for treating or preventing cancer.

According to an aspect of the present invention, the present invention provides a use of an antibody-drug conjugate for preparing a pharmaceutical composition for treating or preventing cancer.

Herein, "treatment" refers to any action whereby the symptoms of the disease are improved or beneficially changed by administering the compound, and "prevention" refers to any action whereby the occurrence, spread, and recurrence of the disease are inhibited or delayed by administering the compound.

Herein, "administration" refers to provision of a given substance to a subject in need thereof by any appropriate method, and the administration route of the antibody-drug conjugate of the present invention may be administered via any general route (*e.g*., oral or parenteral routes) as long as it can reach the target tissue.

Herein, "cancer" comprises any cancer that can be treated by the inhibition of topoisomerase I, and/or inhibition of any one or more cancer-associated survival genes selected from the group consisting of DDX5, survivin, Mcl-1, XIAP, and cIAP2, and may be a solid cancer or hematological cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, paranasal sinus and nasal cavity cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, uretheral cancer, cancer of unknown primary site, gastric lymphoma, gastric cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, pulmonary adenocarcinoma, lung cancer, pulmonary squamous cell carcinoma, skin cancer, anal cancer, rhabdomyosarcoma, laryngeal cancer, pleural cancer, blood cancer, and thymic cancer, but is not limited thereto. Additionally, the cancer comprises not only primary cancer but also metastatic cancer.

According to an aspect of the present invention, the cancer may be one or more selected from the group consisting of liver cancer, colorectal cancer, lung cancer, non-small cell lung cancer, gastric cancer, kidney cancer, and pancreatic cancer.

### Mode for Carrying Out the Invention

### [Examples]

Hereinafter, the constitution of the present invention is specifically described through examples; however, the following examples are only intended to help understanding of the present invention and the scope of the present invention is not limited to these examples. The reagents, solvents, and starting materials described in the examples below, if they are substances that can be easily purchased, their sources of acquisition are not specifically described.

The method for preparing a camptothecin derivative according to the present invention is as follows.

### Preparation Examples: Synthesis of PBX Payload

### Preparation Example 1: Synthesis of Active Camptothecin Derivatives of Formula 2 or Formula 3 (PBX-7011 and PBX-7012)

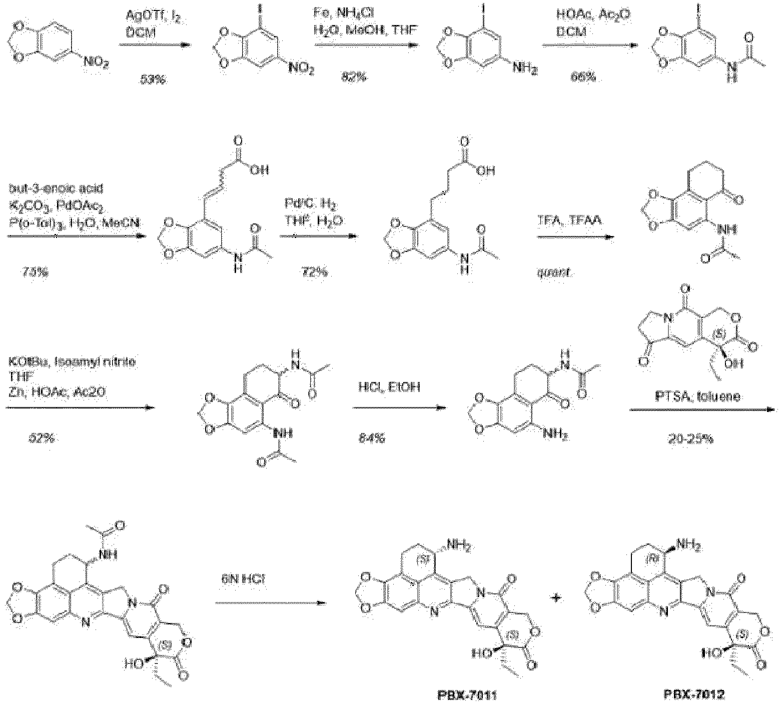

### 1-1. Synthesis of Compound 2

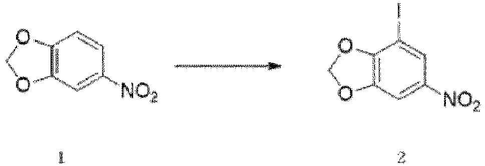

Silver triflate (57.7 g, 224 mmol) and iodine (57.0 g, 224 mmol) were added to a solution of 5-nitrobenzo[d][1,3]dioxole (25 g, 150 mmol) in a dichloromethane (748 mL) flask. The solution was stirred in the dark at room temperature under a N₂ atmosphere.

AgI was removed by filtration and the solid was washed with dichloromethane (100 mL). The solvent was removed under reduced pressure and the residue was partitioned between EtOAc (250 mL) and 5% (v/v) a NH₄OH/H₂O solution (200 mL). The organic layer was separated, washed with 1 M Na₂SO₃ (5 × 200 mL) and brine (200 mL), dried over Na₂SO₄, treated with activated charcoal, and filtered through Celite. The solvent was evaporated under reduced pressure to obtain the crude product as a brown solid. This was triturated in ice-cold EtOH (400 mL), filtered, and the solid was washed with ice-cold EtOH (100 mL) to give the product as a pale brown-gray solid (14.3 g). The solvent was removed from the residue and the crude product was triturated a second time in ice-cold EtOH (300 mL). The solid was recovered by filtration and washed with EtOH (50 mL) to obtain an additional amount of the product (10.2 g) as a dark brown-gray solid. Both batches were used as-is without further purification.

SC_ACID: m/z 294.2 [M+H]⁺

### 1-2. Synthesis of Compound 3

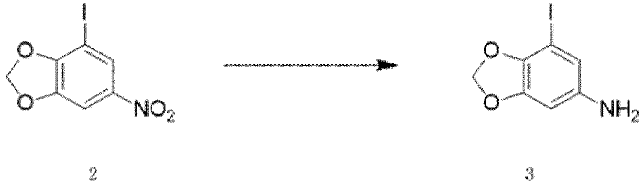

To a suspension of 4-iodo-6-nitrobenzo[d][1,3]dioxole (8.75 g, 29.9 mmol) in a mixture of water (80 mL), methanol (40.0 mL), and tetrahydrofuran (40.0 mL), iron powder (6.67 g, 119 mmol) and ammonium chloride (6.39 g, 119 mmol) were added. The suspension was heated to 75°C and stirred for 16.5 hours. The reaction mixture was cooled to room temperature and concentrated under reduced pressure. The resulting black solid was suspended in ethyl acetate (100 mL) and the solution was decanted. The residue was washed with EtOAc (3 × 50 mL). The mixture was transferred to a separatory funnel, and water was added thereto, and the aqueous layer was removed. The organic layer was washed with saturated aqueous NaHCO₃ solution (150 mL) and brine (150 mL). The organic layer was dried over Na₂SO₄ and the solvent was removed under reduced pressure to obtain a brown solid (4.75 g, 60% yield). An additional product was recovered by thoroughly washing the iron residue with ethyl acetate. The organic fraction was then washed with a saturated aqueous NaHCO₃ solution (150 mL), brine (150 mL), and dried over Na₂SO₄. The solvent was removed under reduced pressure to obtain a brown solid (1.74 g, 22% yield).

SC_ACID: m/z 264.0 [M+H]⁺

### 1-3. Synthesis of Compound 4

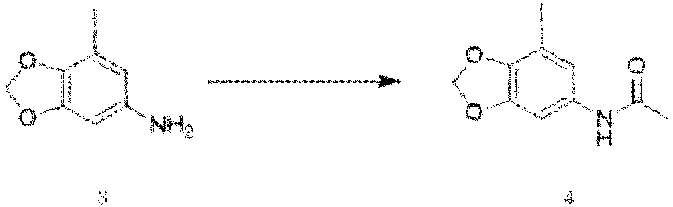

To a solution of 7-iodobenzo[d][1,3]dioxol-5-amine (6.39 g, 24.29 mmol) in dichloromethane (49 mL), acetic anhydride (2.75 mL, 29.2 mmol) and triethylamine (4.06 mL, 29.2 mmol) were added. The reaction mixture was stirred at room temperature overnight. After several hours, additional DCM (10 mL) was added thereto. The suspension was filtered through a sintering funnel and washed with ice-cold DCM (10 mL). The product was further dried under reduced pressure to obtain a light gray solid (4.46 g). The filtrate was concentrated under reduced pressure, and the residue was dissolved in EtOAc, washed with water and brine, dried over Na₂SO₄, and concentrated under reduced pressure to obtain a brown solid (~3 g). The brown solid was purified by flash column chromatography (80 g Si, 0-100% ethyl acetate in heptane). All batches of the product were triturated in ice-cold EtOAc (5-10 mL). The two batches were combined and further dried to obtain an off-white solid.
Total yield: 5.0 g, 66%
SC_ACID: m/z 306.0 [M+H]⁺
¹H NMR (400 MHz, DMSO-d6) δ 9.88 (s, 1H), 7.39 (d, J = 1.9 Hz, 1H), 7.17 (d, J = 1.9 Hz, 1H), 6.04 (s, 2H), 1.99 (s, 3H).

### 1-4. Synthesis of Compound 5

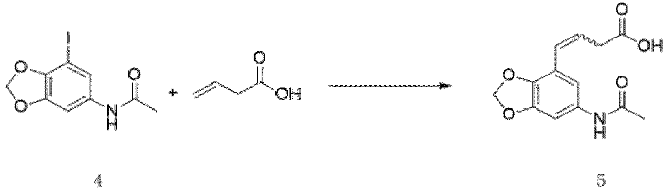

A slurry of N-(7-iodobenzo[d][1,3]dioxol-5-yl)acetamide (5.06 g, 16.59 mmol), *but*-3-enoic acid (1.69 mL, 19.90 mmol), and potassium carbonate (2.98 g, 21.56 mmol) in acetonitrile (40 mL) was cooled to 0°C to 5°C in a 3-neck flask equipped with a condenser. Water (13.33 mL) was slowly added thereto to generate gas. When gas generation stopped, the mixture was degassed with Ar for 30 minutes. Tri-*o-*tolylphosphine (0.505 g, 1.659 mmol) and palladium acetate (0.186 g, 0.829 mmol) were added and the mixture was degassed for another 30 minutes and then heated to reflux under Ar. The reaction mixture was cooled to room temperature and filtered through Celite. The filter cake was washed with H₂O and EtOAc. The organic solvent was removed from the filtrate under vacuum and the aqueous solution was acidified to a concentrated solution. The aqueous layer of HCl was extracted with EtOAc until the pH became 1 to 2, and the combined organic phases were washed with brine, dried over Na₂SO₄, filtered and concentrated under reduced pressure to obtain the crude product. The crude material was triturated in ice-cold EtOAc (30 mL) and the solid was recovered by filtration to obtain the product as a brown solid. The mother liquor was concentrated under reduced pressure and purified by flash chromatography (80 g Si, 0-100% EtOAc in heptane). The product fractions were concentrated under reduced pressure to obtain a light brown foam. A mixture of E/Z isomers was obtained (total yield: 3.46 g, 75%).

SC_ACID: m/z 264.4 [M+H]⁺

### 1-5. Synthesis of Compound 6

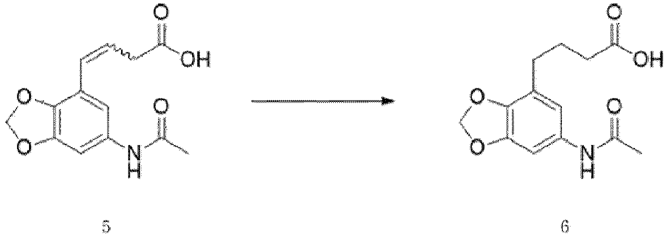

A suspension of 4-(6-acetamidobenzo[d][1,3]dioxol-4-yl)*but*-3-enoic acid (3.47 g, 13.18 mmol) in tetrahydrofuran (50 mL)/water (50 mL) was degassed with N₂ for 15 minutes. Pd/C (2.97 g, 1.397 mmol) was added before heating to 40°C under H₂ (balloon) and the suspension was degassed with H₂ for 5 minutes. After 24 hours, nitrogen and additional Pd/C (2.97 g, 1.397 mmol) were added to the reaction mixture. The reaction mixture was bubbled with hydrogen for 10 minutes and stirred overnight at 45°C under hydrogen. The reaction mixture was filtered through Celite. The filter cake was washed with H₂O (50 mL) and EtOAc (50 mL) and the organic solvent was removed from the filtrate under vacuum. The aqueous solution was acidified with concentrated HCl until pH became 1 and the dark brown/green precipitate was collected by filtration through a sintering funnel. The aqueous filtrate was extracted. The combined organic phases were washed with brine, dried over Na₂SO₄, filtered, and the solvent was removed under vacuum to obtain brown oil. Since the product recovery rate was low, the filter cake was washed with EtOAc (50 mL), water (200 mL), and MeOH (400 mL). The water/EtOAc flush was added to the flask containing the solid from the MeOH flush. The aqueous layer was acidified to a high concentration. An off-white precipitate continued to form until the pH was adjusted to 1 using HCl, and it was collected by filtration through a sintering funnel. After extracting the aqueous filtrate with EtOAc (3 × 200 mL), LCMS showed that all products were removed in the aqueous phase. The combined organic phases were washed with brine, dried over Na₂SO₄, and filtered, and the solvent was removed under vacuum to obtain a light brown solid. All product batches were combined and purified by flash column chromatography (40 g Si, 0-10% MeOH in DCM). The product fractions were concentrated to obtain a light brown solid (Yield: 2.52 g, 72%).

SC_ACID: 266.2 [M+H]⁺
¹H NMR (400 MHz, DMSO-d6) δ 12.07 (s, 1H), 9.78 (s, 1H), 7.14 (d, J = 2.0 Hz, 1H), 6.80 (d, J = 2.2 Hz, 1H), 5.95 (s, 2H), 2.50 - 2.46 (m, 2H), 2.23 (t, J = 7.4 Hz, 2H), 1.98 (s, 3H), 1.84 - 1.70 (m, 2H).

### 1-6. Synthesis of Compound 7

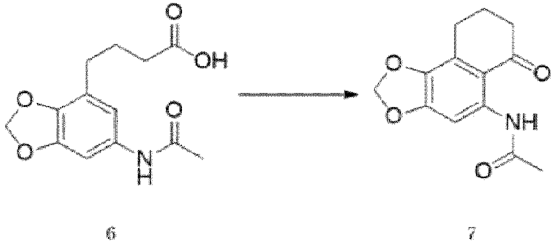

A suspension of 4-(6-acetamidobenzo[d][1,3]dioxol-4-yl)butanoic acid (150 mg, 0.565 mmol) in TFA (433 µL, 5.65 mmol) was cooled on ice. TFAA (157 µL, 1.131 mmol) was added thereto. The mixture was stirred at 0°C to 5°C for 1 hour, and it turned into a dark solution over time. The reaction mixture was added dropwise to an ice-cold saturated aqueous NaHCO₃ solution (10 mL), and the aqueous solution was extracted with ethyl acetate (3 × 25 mL). The saturated NaHCO₃ of combined organic layers and brine were dried over Na₂SO₄, filtered, and the solvent was removed under vacuum to obtain a salmon pink solid (Yield: 140 mg, 100%).

SC_ACID: m/z 248.2 [M+H]⁺
¹H NMR (400 MHz, DMSO-d6) δ 12.34 (s, 1H), 8.11 (s, 1H), 6.13 (s,2H), 2.80 (t, J = 6.2 Hz, 2H), 2.66 - 2.58 (m, 2H), 2.12 (s, 3H), 2.01 - 1.91(m, 2H).

### 1-7. Synthesis of Compound 8

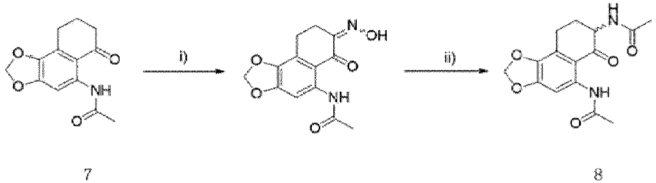

A suspension of N-(6-oxo-6,7,8,9-tetrahydronaphtho[1,2-d][1,3]dioxol-5-yl)acetamide (50 mg, 0.202 mmol) in tetrahydrofuran (1.2 mL) was cooled to 0°C and potassium *tert*-butoxide (27.2 mg, 0.243 mmol) and isoamyl nitrite (35.0 µL, 0.263 mmol) were added thereto. The dark green mixture was stirred on ice (< 5°C) for 1.5 hours. Acetic acid (170 µL, 2.94 mmol), acetic anhydride (170 µL, 1.810 mmol), and zinc dust (66.1 mg, 1.011 mmol) were added to the reaction mixture. The suspension was stirred at 0°C for 2 hours. The reaction mixture was filtered over Celite and flushed with DCM. The filtrate was concentrated under reduced pressure to obtain a black oily substance. The crude product was purified by flash column chromatography (4 g Si, 0-4% MeOH in DCM). The product fractions were concentrated to obtain a gray solid (32 mg, 52%) with 80-90% purity. Higher purity samples can be obtained by preparative MPLC.

SC_ACID: m/z 305.4 [M+H]⁺
¹H NMR (400 MHz, DMSO-d6) δ 12.10 (s, 1H), 8.21 (d, J = 8.0Hz, 1H), 8.12 (s, 1H), 6.15 (d, J = 9.3 Hz, 2H), 4.66 - 4.56 (m, 1H), 2.93(dd, J = 8.9, 4.0 Hz, 2H), 2.14 (s, 3H), 2.13 - 1.93 (m, 2H), 1.91 (s, 3H).

### 1-8. Synthesis of Compound 9

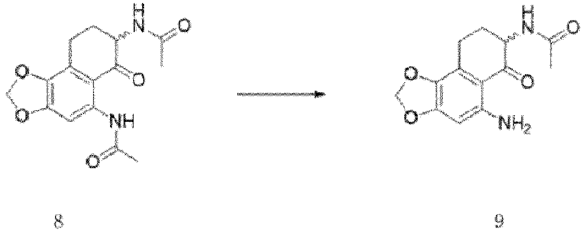

N,N'-(6-Oxo-6,7,8,9-tetrahydronaphtho[1,2-d][1,3]dioxole-5,7-diyl)diacetamide (244 mg, 0.802 mmol) was suspended in 2 M hydrochloric acid (4.69 mL, 9.38 mmol) in ethanol/water (5/1). The mixture was heated to 55°C for 4 hours. The black mixture was cooled to 0°C to 5°C. Triethylamine (1.4 mL, 10.04 mmol) was added dropwise while stirring. The mixture was then diluted with EtOH and evaporated to dryness. The residue was partitioned between water and DCM. The layers were separated and the aqueous layer was extracted once with DCM. The combined organic layers were dried over Na₂SO₄ and concentrated to obtain the product as a brown solid (178 mg, 73%) in 86% purity.

SC_ACID: m/z 263.0 [M+H]⁺
¹H NMR (400 MHz, DMSO) δ 8.04 (d, J = 8.0 Hz, 1H), 6.20 (s, 1H), 5.94 (d, J = 6.0 Hz, 2H), 4.48 - 4.41 (m, 1H), 3.08 (s, 2H), 2.88 - 2.69(m, 2H), 2.15 - 2.03 (m, 1H), 1.88 (s, 3H), 1.86 - 1.75 (m, 1H).

### 1-9. Synthesis of Compound 10

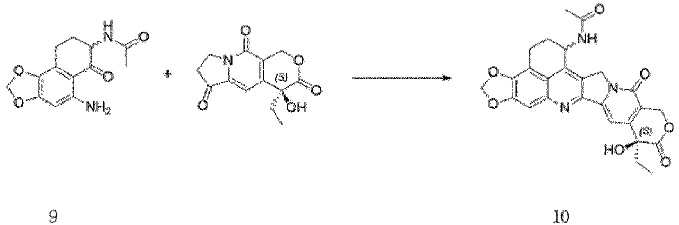

(4S)-4-Ethyl-7,8-dihydro-4-hydroxy-1H-pyrano[3,4-f]indolizin-3,6,10(4H)-trione (146 mg, 0.555 mmol) and N-(5-amino-6-oxo-6,7,8,9-tetrahydronaphtho[1,2-d][1,3]dioxol-7-yl)acetamide (112 mg, 0.427 mmol) were added to dry toluene (4.5 mL). PPTS (21 mg, 0.085 mmol) was added thereto and the reaction mixture was stirred at 115°C for 40 hours.

The reaction mixture was cooled to room temperature. The suspension was diluted with 2 mL DCM and filtered. A black residue (210 mg) was obtained.

The crude product was purified by column chromatography (12 g Si, 0-7% methanol in DCM) to obtain the product (45 mg, 21%) as a brown solid.

LCMS analysis showed two diastereoisomers.

SC_ACID: m/z 490.2 [M+H]⁺
¹H NMR (400 MHz, DMSO-d6) δ 8.47 (t, J = 9.3 Hz, 1H), 7.42 (s, 1H), 7.24 (s, 1H), 6.49 (s, 1H), 6.29 (d, J = 5.2 Hz, 2H), 5.57 - 5.49 (m, 1H), 5.41 (s, 2H), 5.23 - 5.07 (m, 2H), 3.09 - 3.00 (m, 2H), 2.11 - 2.01 (m, 2H), 1.91 (s, 3H), 1.89 - 1.79 (m, 2H), 0.87 (t, J = 7.1 Hz, 3H).

### 1-10. Synthesis of PBX-7011 and PBX-7012

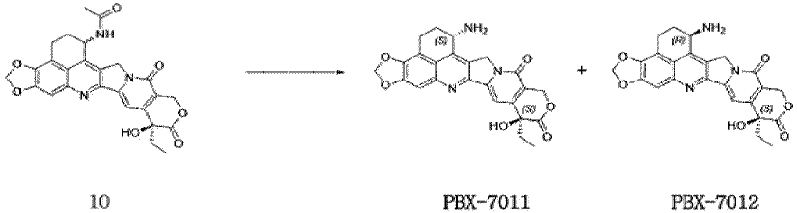

N-((10S)-10-ethyl-10-hydroxy-11,14-dioxo-2,3,10,11,14,16-hexahydro-1H,13H-benzo[de][1,3]dioxolo[4,5-g]pyrano[3',4':6,7]indolizino[1,2-b]quinolin-1-yl)acetamide (73.5 mg, 0.150 mmol) was dissolved in 1.0 mL 6 N HCl and stirred at 90°C for 8 hours and then at room temperature overnight. The reaction mixture was concentrated under reduced pressure and purified by acidic preparative MPLC (Luna2-30) run twice. The fractions comprising the separated diastereomers were acidified with 5 drops of 3 N HCl and lyophilized to obtain the product as a yellow solid.
1st eluting isomer: PBX-7011, 23 mg, 34% yield
U_AN_ACID: m/z 448.4 [M+H]⁺
¹H NMR (400 MHz, DMSO-d6) δ 8.54 (s, 3H), 7.50 (s, 1H), 7.27 (s, 1H), 6.52 (s, 1H), 6.34 (d, J = 13.3 Hz, 2H), 5.77 (d, J = 19.3 Hz, 1H), 5.44(s, 2H), 5.37 (d, J = 19.3 Hz, 1H), 5.05 (s, 1H), 3.19 - 3.02 (m, 2H), 2.46(s, 1H), 2.20 - 2.03 (m, 1H), 1.94 - 1.81 (m, 2H), 0.88 (t, J = 7.3 Hz, 3H).
2nd eluting isomer: PBX-7012, 28 mg, 41% yield
U_AN_ACID: m/z 448.2 [M+H]⁺
¹H NMR (400 MHz, DMSO-d6) δ 8.57 (d, J = 4.7 Hz, 3H), 7.51 (s, 1H), 7.27 (s, 1H), 6.52 (s, 1H), 6.34 (d, J = 12.2 Hz, 2H), 5.76 (d, J = 19.4 Hz, 1H), 5.44 (s, 2H), 5.37 (d, J = 19.4 Hz, 1H), 5.08 (s, 1H), 3.16 - 2.98(m, 2H), 2.46 (s, 1H), 2.18 - 2.06 (m, 1H), 1.94 - 1.80 (m, 2H), 0.87 (t, J = 7.3 Hz, 3H).

### Preparation Example 2: Synthesis of Active Camptothecin Derivatives of Formula 4 or Formula 5 (PBX-7014 and PBX-7015)

This Example describes the synthesis of compounds PBX-7014 and PBX-7015 starting from two separated diastereomers of the Exatecan-hybrid compounds (PBX-7011 and PBX-7012).

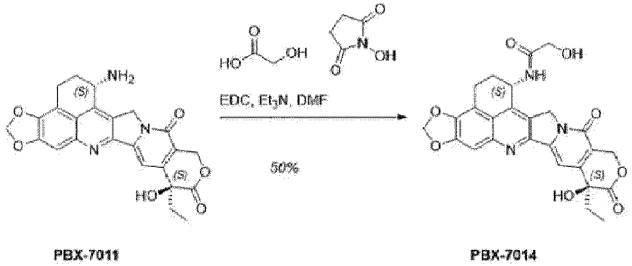

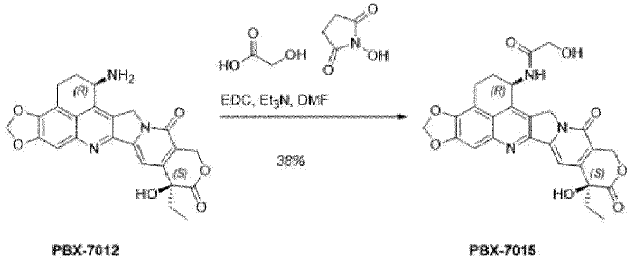

### 2-1: Synthesis of PBX-7014

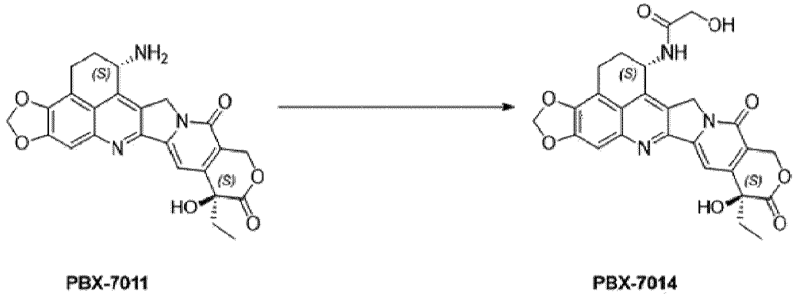

A stock solution of an activated glycolic acid was prepared according to the following procedure:
Glycolic acid (17 mg, 0.224 mmol) was dissolved in 1 mL of N,N-dimethylformamide. HOSu (25.7 mg, 0.223 mmol) and EDC (42.8 mg, 0.223 mmol) were added thereto. The reaction mixture was stirred at room temperature for 1 hour.

Next, 0.4 mL of the activated acid solution was added to a suspension of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and triethylamine (0.025 mL, 0.179 mmol) in N,N-dimethylformamide (2.5 mL). The mixture was stirred at room temperature for 3 hours. 0.05 mL of a freshly prepared activated acid solution was added thereto. Then, the reaction mixture was stirred at room temperature for an additional 2 hours. The reaction mixture was evaporated to dryness. The crude product was purified by column chromatography (0-8% methanol in chloroform). This gave a yellow solid which comprises the PBX-7014 product and residual succinimide. The product was further purified by acid preparative MPLC (Luna5-40) and the product fractions were lyophilized to obtain a light yellow solid. (Yield: 20 mg, 50%)

U_AN_ACID: m/z 506.2 [M+H]⁺
¹H NMR (400 MHz, DMSO-d6) δ 8.40 (d, J = 8.9 Hz, 1H), 7.40 (s, 1H), 7.23 (s, 1H), 6.49 (s, 1H), 6.28 (d, J = 4.6 Hz, 2H), 5.61 - 5.45 (m, 2H), 5.45 - 5.35 (m, 2H), 5.19 - 5.06 (m, 2H), 3.95 (s, 2H), 3.13 - 2.96 (m, 2H), 2.21 - 2.02 (m, 2H), 1.94 - 1.77 (m, 2H), 0.87 (t, J = 7.3 Hz, 3H).

### 2-2: Synthesis of PBX-7015

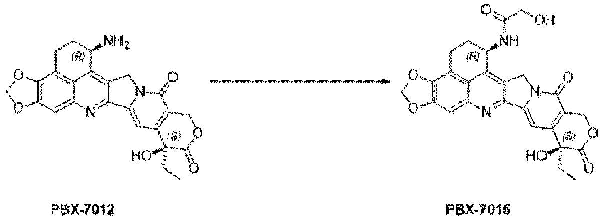

A stock solution of an activated acid was prepared according to the following procedure:
Glycolic acid (17.00 mg, 0.223 mmol) was dissolved in 1 mL of N,N-dimethylformamide. HOSu (25.7 mg, 0.223 mmol) and EDC (42.8 mg, 0.223 mmol) were added thereto. The reaction mixture was stirred at room temperature for 1 hour.

Next, 0.25 mL of the activated acid solution was added to a suspension of (1R,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (25 mg, 0.056 mmol) and triethylamine (0.016 mL, 0.112 mmol) in N,N-dimethylformamide (2.5 mL). The mixture was stirred at room temperature overnight. 0.03 mL of a freshly prepared activated acid solution was added thereto. The reaction mixture was then stirred at room temperature for 2 hours. The reaction mixture was combined with the previous smaller batch and evaporated to dryness. The crude product was purified by column chromatography. This gave a yellow solid comprising the PBX-7015 product and residual succinimide. The product was purified by acid preparative MPLC (Luna5-40). The product fractions were lyophilized to obtain a light yellow solid. (Yield: 15 mg, 38%)

U_AN_ACID: 506.2 [M+H]⁺
¹H NMR (400 MHz, DMSO) δ 8.44 (d, J = 9.0 Hz, 1H), 7.41 (s, 1H), 7.24 (s, 1H), 6.48 (s, 1H), 6.29 (d, J = 2.3 Hz, 2H), 5.61 - 5.44 (m, 2H), 5.44 - 5.36 (m, 2H), 5.20 - 5.07 (m, 2H), 3.96 (s, 2H), 3.10 - 2.96 (m, 2H), 2.20 - 2.06 (m, 2H), 1.95 - 1.79 (m, J = 7.3 Hz, 2H), 0.87 (t, J = 7.3 Hz, 3H).

### Preparation Example 3: Synthesis of Active Camptothecin Derivative of Formula 6 or 7 (PBX-7016 and PBX-7017)

This Example describes the synthesis of Compound PBX-7016 starting from an Exatecan-hybrid compound (PBX-7011).

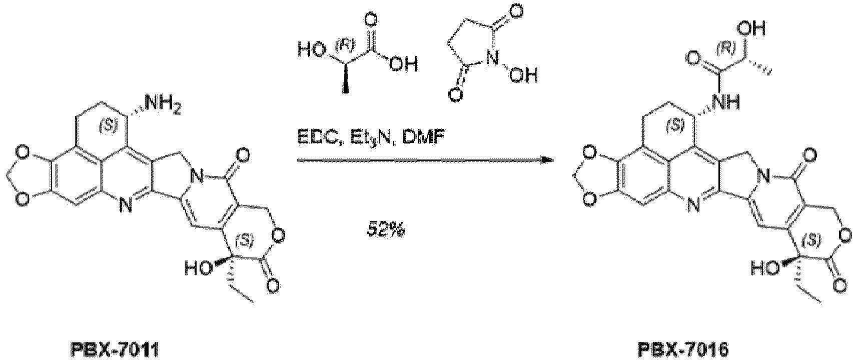

A stock solution of the activated D-lactic acid was prepared according to the following procedure.

D-Lactic acid (22 mg, 0.244 mmol) was dissolved in 1 mL of N,N-dimethylformamide. HOSu (27 mg, 0.235 mmol) and EDC (38.6 mg, 0.201 mmol) were added thereto. The reaction mixture was stirred at room temperature for 2 hours.

Next, 0.3 mL of the activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (36 mg, 0.080 mmol) and triethylamine (0.022 mL, 0.161 mmol) in N,N-dimethylformamide (2.5 mL). The mixture was stirred at room temperature for 6 hours. 0.05 mL of the prepared activated acid solution was added thereto. Then, the reaction mixture was stirred at room temperature overnight. The reaction mixture was purified directly by acid preparative MPLC (Luna10-50), and the product fractions were lyophilized to obtain a light yellow solid.
Yield: 22mg, 52%
U_AN_ACID: m/z 520.2 [M+H]⁺
¹H NMR (400 MHz, DMSO) δ 8.43 (d, J = 9.1 Hz, 1H), 7.41 (s, 1H), 7.23 (s, 1H), 6.50 (s, 1H), 6.29 (d, J = 2.1 Hz, 2H), 5.62 - 5.58 (m, 1H), 5.58 - 5.51 (m, 1H), 5.41 (s, 2H), 5.21 - 5.01 (m, 2H), 4.17 - 4.07 (m, 1H), 3.14 - 2.95 (m, 2H), 2.19 - 2.04 (m, 2H), 1.92 - 1.78 (m, 2H), 1.39 (d, J = 6.8 Hz, 3H), 0.87 (t, J = 7.3 Hz, 3H).

As described above, since PBX-7016 of Formula 6 can be synthesized from PBX-7011 of Formula 2, PBX-7017 of Formula 7 can be synthesized from PBX-7012 of Formula 3 in the same manner.

### Preparation Example 4: Synthesis of Active Camptothecin Derivative of Formula 8 (PBX-7018)

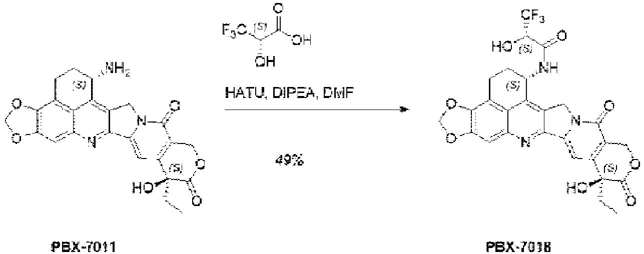

(S)-3,3,3-Trifluoro-2-hydroxypropanoic acid (11.59 mg, 0.080 mmol) was dissolved in N,N-dimethylformamide (2 mL) and hexafluorophosphate azabenzotriazole tetramethyl uronium (HATU) (30.6 mg, 0.080 mmol) was added thereto.

After the mixture was stirred for 3 minutes, (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (which was named PBX-7011) (30 mg, 0.067 mmol) and N,N-Diisopropylethylamine (DIPEA) (0.047 mL, 0.268 mmol) were added thereto, and the reaction mixture was stirred at room temperature for 1.5 hours. The reaction mixture was purified directly by acid preparative MPLC (Luna10-50) and the product fractions were lyophilized to obtain the product as an off-white solid. (Yield: 19 mg, 49%)

The PBX-7011 can be obtained by the method described in PCT/KR2023/009854, *etc.,* but the method is not limited thereto.

U_AN_ACID: m/z 574.2 [M+H]⁺
¹H NMR (400 MHz, DMSO-d6) δ 8.95 (d, *J* = 8.6 Hz, 1H), 7.42 (s, 1H), 7.24 (s, 1H), 7.18 (d, *J* = 6.2 Hz, 1H), 6.48 (s, 1H), 6.29 (d, *J =* 7.1 Hz, 2H), 5.61 - 5.52 (m, 1H), 5.46 - 5.35 (m, 2H), 5.21 (d, *J=* 19.2 Hz, 1H), 5.06 (d, *J=* 19.2 Hz, 1H), 4.66 - 4.55 (m, 1H), 3.08 - 2.97 (m, 2H), 2.18 - 2.03 (m, 2H), 1.93 - 1.77 (m, 2H), 0.87 (t, *J=* 7.3 Hz, 3H).

### Preparation Example 5: Synthesis of Active Camptothecin Derivative of Formula 9 (PBX-7020)

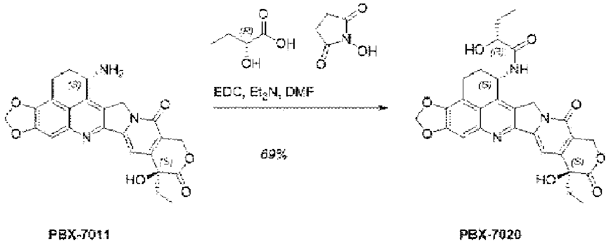

(R)-2-Hydroxybutyric acid (20.9 mg, 0.201 mmol) was dissolved in 1 mL DMF. HOSu (23.1 mg, 0.201 mmol) and EDC (38.6 mg, 0.201 mmol) were added, and the reaction mixture was stirred at room temperature for 2 hours.

Next, 0.3 mL of the activated acid solution was added to a suspension of PBX7011 (30 mg, 0. 067 mmol) and triethylamine (0.019 ml, 0.134 mmol) in N,N-dimethylformamide (2 mL). The mixture was stirred at room temperature for 23 hours. The reaction mixture was purified directly by acidic preparative MPLC (Luna10-50), which gave an off-white solid after lyophilization of the product fractions. (Yield: 25 mg, 69%)
U_AN_ACID: m/z 534.2 [M+H]⁺
¹H NMR (400 MHz, DMSO) δ 8.39 (d, J = 8.9 Hz, 1H), 7.41 (s, 1H), 7.23 (s, 1H), 6.48 (s, 1H), 6.29 (d, J = 3.6 Hz, 2H), 5.57 - 5.50 (m, 1H), 5.48 - 5.44 (m, 1H), 5.41 (s, 2H), 5.13 (s, 2H), 3.92 - 3.86 (m, 1H), 3.06 - 3.00 (m, 2H), 2.15 - 2.05 (m, 2H), 1.92 - 1.74 (m, 3H), 1.71 - 1.62 (m, 1H), 0.93 (t, J = 7.4 Hz, 3H), 0.87 (t, J = 7.3 Hz, 3H).

### Preparation Example 6: Synthesis of Active Camptothecin Derivative of Formula 10 (PBX-7022)

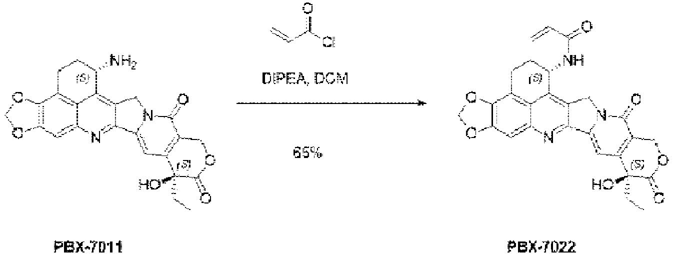

A suspension of PBX-7011 (30 mg, 0. 067 mmol) and DIPEA (0.047 mL, 0.268 mmol) in dichloromethane (dry) (1 mL) was cooled to 0°C. A solution of acryloyl chloride (5.44 µL, 0.067 mmol) was added dropwise to 0.1 mL of DCM. The reaction mixture was stirred at 0°C, and after 30 minutes, 0.15 equivalents of acrylonitrile chloride was added to stock solution of DCM. After stirring for a total of 2 hours, the reaction mixture was concentrated under reduced pressure. The residue was dissolved in DMSO and purified by acid preparative MPLC (Reprosil 10-50 method). The product fractions were collected and lyophilized to obtain a light yellow fluffy solid. (Yield: 22 mg, 65%)

U_AN_ACID: m/z 502.2 [M+H]⁺
¹H NMR (400 MHz, DMSO-d6) δ 8.71 (d, *J* = 8.8 Hz, 1H), 7.43 (s, 1H), 7.23 (s, 1H), 6.49 (s, 1H), 6.30 (d, *J* = 4.2 Hz, 2H), 6.28 - 6.21 (m, 2H), 5.69 (dd, *J* = 8.5, 3.7 Hz, 1H), 5.65 - 5.58 (m, 1H), 5.40 (s, 2H), 5.25 - 5.12 (m, 1H), 5.12 - 4.99 (m, 1H), 3.10 - 3.02 (m, 2H), 2.16 - 2.06 (m, 2H), 1.92 - 1.78 (m, 2H), 0.86 (t, *J* = 7.3 Hz, 3H).

### Preparation Example 7: Synthesis of Active Camptothecin Derivative of Formula 11 (PBX-7024)

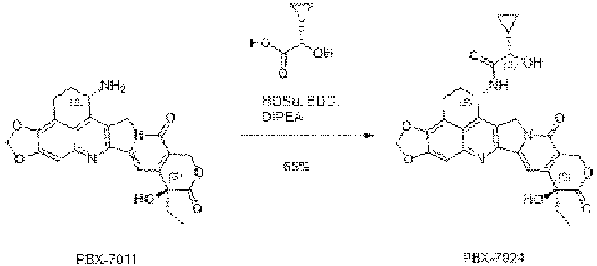

PBX-7024 was prepared in high yield by linking (2S)-2-cyclopropyl-2-hydroxyacetic acid to the PBX-7011 compound.

(2S)-2-Cyclopropyl-2-hydroxyacetic acid (26 mg, 0.244 mmol) was dissolved in 1 mL of N,N-dimethylformamide. HOSu (26 mg, 0.226 mmol) and EDC (42 mg, 0.219 mmol) were added thereto. The reaction mixture was stirred at room temperature for 2 hours. Next, 0.6 mL of the activated acid solution was added to a solution of (1S,10S)-1-amino-10-ethyl-10-hydroxy-1,2,3,10,13,16-hexahydro-11H,14H-benzo[de][1,3]dioxolo[4,5-g]pyrano[3',4':6,7]indolizino[1,2-b]quinolin-11,14-dione (40 mg, 0.089 mmol) and DIPEA (0.047 mL, 0.268 mmol) in N,N-dimethylformamide (2.5 mL). The mixture was stirred at room temperature overnight. The reaction mixture was purified directly by acidic preparative MPLC (Luna10-50), and the product fractions were lyophilized to obtain a bright white solid.
Yield: 32 mg, 65%
U_AN_ACID: m/z 520.2 [M+H]⁺
¹H NMR (400 MHz, DMSO-d6) δ 8.33 (d, J = 8.7 Hz, 1H), 7.39 (s, 1H), 7.23 (s, 1H), 6.48 (s, 1H), 6.28 (d, J = 4.9 Hz, 2H), 5.50 (q, J = 6.7 Hz, 1H), 5.40 (s, 3H), 5.23 - 5.06 (m, 2H), 3.62 (d, J = 6.3 Hz, 1H), 3.03 (q, J = 6.2 Hz, 2H), 2.21 - 2.02 (m, 2H), 1.92 - 1.79 (m, 2H), 1.18 - 1.08 (m, 1H), 0.87 (t, J = 7.3 Hz, 3H), 0.47 - 0.30 (m, 4H).

### Preparation Example 8: Synthesis of Linker-Payload Compound (mc-GGFG-PBX-7016)

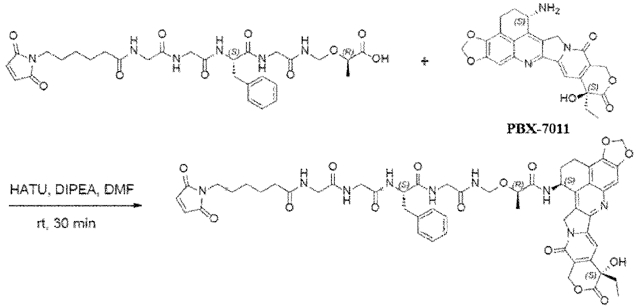

PBX-7011 (410 mg, 754 µmol) and (2R,10S)-10-benzyl-23-(2,5-dioxo-2,5-dihydro-1H-pyrrol-1-yl)-2-methyl-6,9,12,15,18-penta-oxo-3-oxa-5,8,11,14,17-pentaazatricosanoic acid (466 mg, 739 µmol), which has the mc-ggfg structure, were dissolved in 10 mL of N,N-dimethylformamide. DIPEA (439 mg, 3.39 mmol) and HATU (430 mg, 1.13 mmol) were dissolved successively and stirred at room temperature for 30 minutes. Then, the mixture was purified 3 times by acid preparative MPLC (Luna10-50), and the mc-GGFG-PBX-7016 compound was obtained as a light yellow solid through lyophilization.
Yield: 510 mg, 63%
U_AN_ACID: m/z 1060.02 [M+H]⁺
¹H NMR (400 MHz, DMSO-d6) δ 8.62 (t, J = 6.6 Hz, 1H), 8.50 (d, J = 9.1 Hz, 1H), 8.29 (t, J = 5.9 Hz, 1H), 8.13 - 7.96 (m, 3H), 7.39 (s, 1H), 7.27 - 7.12 (m, 6H), 6.99 (s, 2H), 6.48 (s, 1H), 6.26 (d, J = 6.0 Hz, 2H), 5.61 - 5.51 (m, 1H), 5.45 - 5.33 (m, 2H), 5.20 - 4.99 (m, 2H), 4.72 - 4.63 (m, 1H), 4.57 - 4.41 (m, 2H), 4.11 (q, J = 6.7 Hz, 1H), 3.78 - 3.53 (m, 6H), 3.40 - 3.34 (m, 2H), 3.16 - 2.95 (m, 3H), 2.74 (dd, J = 13.8, 9.5 Hz, 1H), 2.17 - 2.03 (m, 4H), 1.91 - 1.78 (m, 2H), 1.52 - 1.35 (m, 7H), 1.25 - 1.12 (m, 2H), 0.87 (t, J = 7.3 Hz, 3H).

### Examples: Anti-c-Met Antibody-Drug Conjugates (ADC) Comprising Compound of Formula 1 (PBX Payload)

Anti-c-Met ADCs, which comprise the PBX payload prepared in Preparation Examples above, were prepared (Table 1).

**[Table 1]**

| No. | Payload | Linker | Antibody |
|---|---|---|---|
| Example 1 | PBX-7011 | | |
| Example 2 | PBX-7014 | | |
| Example 3 | PBX-7016 | | anti-c-Met antibody (M01) |
| Example 4 | PBX-7018 | mc-GGFG | |
| Example 5 | PBX-7020 | | |
| Example 6 | PBX-7022 | | |
| Example 7 | PBX-7024 | | |

Linker-payloads, which comprise a PBX payload and onto which GGFG (*i.e*., an enzymatically cleavable linker system) is introduced, were synthesized. Specifically, as the linker-payload, those which have the molecular structure of an mc-GGFG-PBX payload and a structure of Formula 13 as a linker were used.

The linker was first reacted with the payload to create a linker-payload conjugate. For example, the linker-payloads comprised in Example 3 were synthesized by the method described in Preparation Example 8 above.

Furthermore, ADCs were synthesized by conjugating with an anti-c-Met antibody (M01) having CDRs of SEQ ID NOs: 1 to 6. The ADCs were prepared by the following method. The prepared c-Met antibody was buffer-exchanged with a reaction buffer (150 mM NaCl, 50 mM histidine, pH 6.0) using a NAP-5 column (Cytiva) or PD-10 desalting column (Cytiva), and then treated the antibody with 30 equivalents of 50 mM TCEP (Tris(2-carboxyethyl)phosphine hydrochloride) (Merck) at 25°C for 2 hours to generate thiol sites necessary for the reaction from the disulfide of the antibody.

Afterward excess TCEP was removed using a NAP-5 column or PD-10 desalting column, and was exchanged with reduction buffer (25 mM Histidine, pH 6.0). The conjugation reaction was performed by reacting 12 equivalents of the 5 mM linker-payload compounds prepared above with reduced c-Met antibody at 4°C for 1 hour. As a result, ADCs were prepared in which linker-payload compound was conjugated to an anti-c-Met antibody.

### Evaluation of Antibody-Drug Conjugates (ADCs)

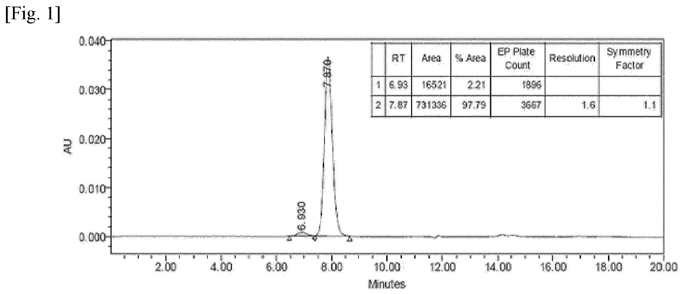

The purity of the anti-c-Met ADC monomers of Example 3 was confirmed using size exclusion chromatography (SEC) and the results are shown in Fig. 1.

The DAR was evaluated by the analysis of mass (Da) and area via LC-MS and the results are shown in Table 2.

**[Table 2]**

| Example 3 | | Mass (Da) | Area | Drug-Based Calculation | Ratio | | DAR |
|---|---|---|---|---|---|---|---|
| Light Chain | D0 | 23814.0 | - | - | - | 1.0 | 8.0 |
| | D1 | 24874.9 | 326425.9 | 326425.9 | 100.0 | | |
| | D2 | - | - | - | - | | |
| Heavy Chain | D0 | 50538.2 | - | - | - | 3.0 | |
| | D1 | 51598.1 | - | - | - | | |
| | D2 | 52658.0 | - | - | - | | |
| | D3 | 53718.9 | 154691.0 | 154691.0 | 100.0 | | |

### Comparative Example: Preparation of Isotype ADC and Anti-c-Met ADC with a different Payload

ADCs, in which the anti-c-Met antibody (M01) is conjugated by having each of the known camptothecin and monomethyl auristatin E (MMAE) as a payload, were prepared using the methods described in Examples above. Additionally, ADCs, in which the anti-SARS-CoV-2 antibody (P63) or anti-influenza A antibody (Navivumab, P23) is conjugated by having PBX-7016 as a payload, were prepared using the method described in Examples above. Finally, ADCs, in which the anti-influenza A antibody (Navivumab, P23) is conjugated by having monomethyl auristatin E (MMAE) as a payload, were prepared. The anti-SARS-CoV-2 antibody (P63) is an antibody which comprises a heavy chain with an amino acid sequence of SEQ ID NO: 11 and a light chain with an amino acid sequence of SEQ ID NO: 12. The anti-influenza A antibody (navivumab, P23) is an antibody which comprises a heavy chain with an amino acid sequence of SEQ ID NO: 13 and a light chain with an amino acid sequence of SEQ ID NO: 14.

Camptothecin was synthesized using a compound of Formula I (the structure comprising the linker is Formula V) disclosed in US 2022/0378935, and MMAE was purchased from MedChemExpress in the form of a payload and a linker-payload (mc-vc-PAB-MMAE). The ADC having PBX-7016 as a payload was used in the form of a linker-payload (mc-GGFG-PBX-7016) using mc-GGFG as a linker.

The SEC and DAR values for each ADC above are as shown in Table 3 below.

**[Table 3]**

| | ADC | SEC | DAR |
|---|---|---|---|
| 1 | M01-Camptothecin | >97% | 6.3 |
| 2 | M01-MMAE | >97% | 3.5 |
| 3 | P63-PBX-7016 | >97% | 8 |
| 4 | P23-PBX-7016 | >97% | 8 |
| 5 | P23-MMAE | >97% | 3.1 |

### Experimental Example 1: Cell Viability Assay of PBX Payload Anti-c-Met ADC

### Experimental Example 1-1. Selection of c-Met Expressing Cell Lines

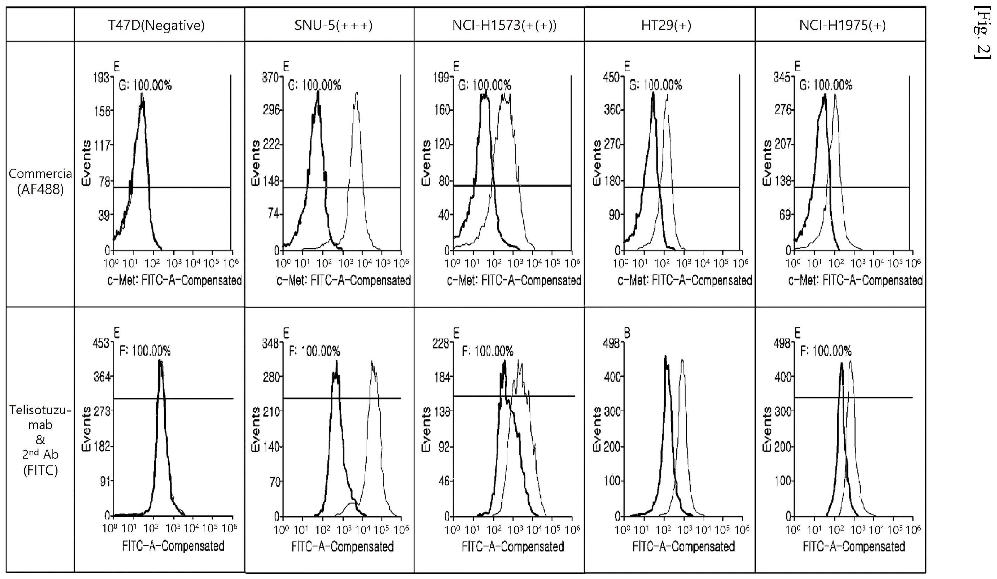

Cell lines expressing c-Met were selected using flow cytometry. The cell lines used were T47D (ATCC), SNU-5 (KCLB), H1573 (KCLB), HT29 (KCLB), and H1975 (Elab science), and antibody binding affinity and flow cytometry analysis were performed using commercially available monoclonal antibodies (FITC-mAb, 11-8858-42) and anti-c-MET antibody (M01), and FITC-anti human IgG Fc (410720) (Fig. 2).

The highest level of c-Met expression was observed in SNU-5 (a gastric cancer cell line), and mean fluorescence intensity (MFI) and the staining indexes in each cell line are as shown in Table 4 below.

**[Table 4]**

| Cell Line | Tissue (Cancer Type) | MFI | | Staining Index | Expression |
|---|---|---|---|---|---|
| | | No Staining | Conjugated mAb | | |
| T47D | Breast (ductal) | 9 | 11 | 1 | - |
| SNU-5 | Stomach (gastric) | 42 | 5,995 | 143 | +++ |
| NCI-H1573 | Lung (adenocarcinoma) | 19 | 549 | 29 | +(+) |
| HT29 | Colon (adenocarcinoma) | 14 | 162 | 12 | + |
| NCI-H1975 | Lung (adenocarcinoma) | 12 | 139 | 12 | + |

### Experimental Example 1-2. Cell Viability Assay

An *in-vitro* cell viability assay was performed on the cell lines selected in Experimental Example 1-1 using the ADCs prepared in Example 3 and Comparative Example above, PBX-7016 payload compound, and MMAE payload compound.

Specifically, 3,000 cell lines listed in Table 4 were seeded per well in a 96-well plate and cultured at constant temperature (37°C, 5% CO₂). After 24 hours, the cells were treated with 100 µL of the payload compound or ADC drugs at 9 concentrations (a 5-fold serial dilution starting from 1,000 nM). In particular, a control group (drug concentration 0), which is an untreated group, was also prepared. After 6 days of incubation at constant temperature (37°C, 5% CO₂), 100 µL of CellTiter-Glo reagent (CellTiter-Glo^{®} Luminescent Cell Viability Assay kit (Promega, G7571)) was added to each well and pipetted. Fluorescence was measured after incubation at constant temperature (RT) for 10 minutes. When the fluorescence value at 0 drug concentration is considered 100%, the concentration that shows 50% of the fluorescence value was measured as the IC₅₀ value.

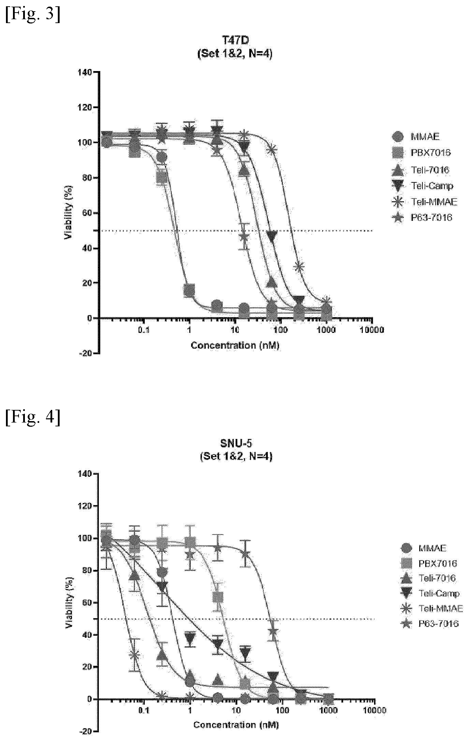

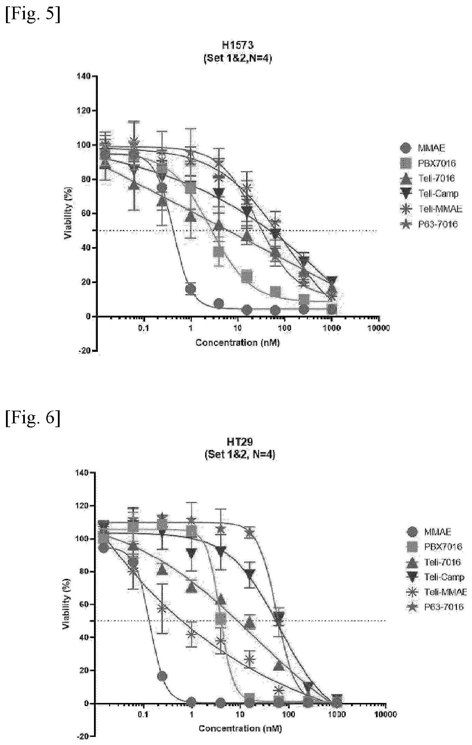

As a result, it was confirmed that the ADC of Example 3 (anti-c-Met ADC) had excellent cell inhibition ability. The specific IC₅₀ values (unit: nM) for each ADC are as shown in Table 5 below (see Figs. 3 to 7).

**[Table 5]**

| Cell Line | MMAE | PBX-7016 | M01-PBX-7016 (Example 3) | M01-Camptothec in | M01-MMAE | P63-PBX-7016 (Isotype ADC) |
|---|---|---|---|---|---|---|
| T47D | 0.51 | 0.45 | 32.6 | 58.2 | 169 | 14.6 |
| SNU-5 | 0.42 | 5.21 | 0.11 | 0.9 | 0.04 | 54.5 |
| NCI-H1573 | 0.42 | 2.55 | 6.1 | 47.5 | 65 | 32.5 |
| HT29 | 0.14 | 3.8 | 8.7 | 54.5 | 0.9 | 61.4 |
| NCI-H1975 | 0.3 | 9.8 | 182.1 | 283.7 | 31.7 | 97.6 |

Specifically, in the SNU-5 cell line where c-Met is expressed in large amounts, it was observed that the anti-c-Met ADC of Example 3 had an IC₅₀ value that was about 50 times superior to the PBX-7016 compound itself and about 500 times superior to the isotype ADC. Improved IC₅₀ values compared to the isotype ADC (P63-PBX-7016) were also shown in c-Met low-expressing cell lines (NCI-H1573 and HT29), thus confirming that when PBX-7016 is developed as an ADC, it can exhibit a particularly excellent inhibitory effect against target cell lines that express a large amount of c-Met. In addition, the anti-c-Met ADC of Example 3 was observed to have a superior IC₅₀ value compared to the competitive drug M01-camptothecin ADC, in which another camptothecin-based payload was conjugated to an anti-c-Met antibody, for all tested cell lines, thus confirming the excellent inhibitory effect of the anti-c-Met ADC of Example 3 against the target cell lines.

Accordingly, the ADC of the present invention, which has a camptothecin derivative compound of Formula 1 as a payload and is specific for c-Met proteins, exhibits an excellent inhibitory effect on cells expressing c-Met, and thus can be used for treatment or prevention of cancer.

### Experimental Example 2. c-Met binding assay of PBX payload anti-c-Met ADC

In preparing an ADC by conjugating a linker and a payload to an anti-c-Met antibody, the effect on binding affinity was investigated. For this purpose, an assay for c-Met was performed using the anti-c-Met ADC of Example 3 of Table 1 and the anti-c-Met antibody (M01) constituting the same.

### ELISA assay

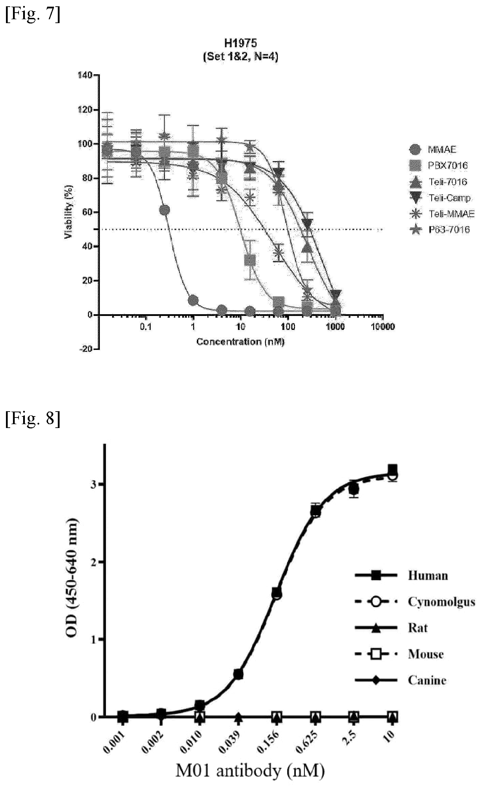

First, a binding assay of M01 antibody alone was performed. Specifically, it was attempted to confirm the binding affinity of M01 antibody for c-Met proteins derived from human, mice, rats, dogs, and monkeys through ELISA assay. First, the M01 antibody was diluted and cross-reactivity tests for said c-Met proteins derived from various species were performed through ELISA assay. As a result, it was confirmed that the M01 antibody specifically binds to recombinant human c-Met and monkey c-Met, but does not bind to c-Met derived from mice, rats, and dogs (Fig. 8).

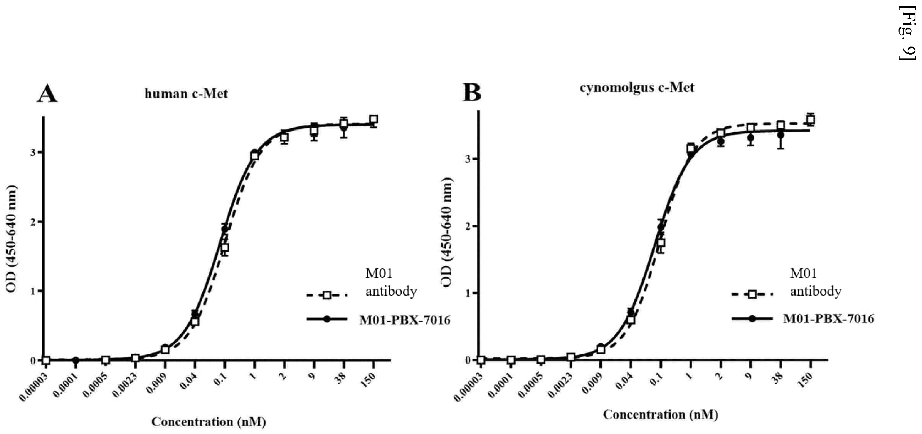

Based on the results above, ELISA assays were performed for the anti-c-Met ADC and M01 antibody of Example 3 against recombinant human c-Met and monkey c-Met (cynomolgus c-Met). As a result, similar EC₅₀ values were confirmed in the anti-c-Met ADC and M01 antibodies, and the specific values are shown in Table 6 below (Fig. 9).

**[Table 6]**

| EC₅₀ (nM) | | M01-PBX-7016 (Example 3) | M01 Antibody |
|---|---|---|---|
| Origin of c-Met Antigen | Monkey | 0.11 | 0.14 |
| | Human | 0.12 | 0.14 |

As a result of the *in-vitro* experiments above, it was confirmed that both the anti-c-Met ADC and M01 antibody of Example 3 bind to recombinant human and monkey c-Met proteins in a concentration-dependent manner.

Specifically, the EC₅₀ values were confirmed to be 0.11 nM and 0.14 nM for monkey-derived c-MET protein, and 0.12 nM and 0.14 nM for human c-MET protein. As such, it was confirmed that the binding characteristics did not change even when the M01 antibody was bound to the linker and the payload in the anti-c-Met ADC of the present invention.

### BLI assay

Subsequently, a biolayer interferometry (BLI) analysis was performed to determine the binding affinity of the anti-c-Met ADC and M01 antibody of the present invention for c-Met proteins.

The binding affinity constants (KD) of the anti-c-Met ADC of Example 3 and M01 antibody for human and monkey-derived c-Met proteins are as shown in Table 7 below.

These results also showed that the binding affinity of the anti-c-Met ADC of the present invention and the M01 antibody for human-derived c-Met protein were similar, thus confirming that the binding affinity of the ADC was maintained even after conjugation. Similarly, the anti-c-Met ADC and M01 antibody of the present invention against the monkey-derived c-Met protein also showed binding affinity similar to the human-derived c-Met protein.

**[Table 7]**

| Origin of c-Met Protein | Loading Sample | KD (nM) | Kₒₙ (1/Ms) | K_{dis} (1/s) | Full R² |
|---|---|---|---|---|---|
| Human | M01-PBX-7016 (Example 3) | 0.774 | 8.13E+04 | 6.29E-05 | 0.9421 |
| | M01 Antibody | 0.605 | 6.74E+04 | 4.07E-05 | 0.9518 |
| Monkey | M01-PBX-7016 (Example 3) | 0.917 | 1.00E+05 | 9.17E-05 | 0.9608 |
| | M01 Antibody | 0.707 | 8.05E+04 | 5.69E-05 | 0.9587 |

### CELISA and Immunofluorescence Staining

Finally, an attempt was made to confirm the binding of the anti-c-Met ADC of the present invention and M01 antibody to c-Met protein expressed on the surface of tumor cells. This was analyzed based on cell-based ELISA (CELISA) and the expression levels of surface c-Met proteins of cell lines for each cancer type.

The quantification of c-Met proteins on the cell surface was assessed by indirect immunofluorescence staining of cell surface antigens. The cell lines used in the present invention have various expression levels of c-Met proteins. This is quantified as c-Met antigen binding capacity (ABC), which represents the number of cell surface c-Met protein molecules, and is expressed as transcripts per kilobase million (TPM) value.

As shown in Table 8 below, TPM values and cell surface c-Met protein expression levels showed a significant correlation.

**[Table 8]**

| Cell Lines | cMet Expression (binding sites/cell) | TPM Values |
|---|---|---|
| Lung Cancer | | |
| EBC-1 | ~ 216,000 | 2633.62 |
| NCI-H1373 | ~ 69,000 | N/A |
| HCC-827 | ~ 53,000 | 310.45 |
| NCI-H1975 | ~ 24,000 | 230.67 |
| NCI-H1573 | ~ 14,000 | 439.89 |

| Gastric Cancer | | |
|---|---|---|
| SNU-5 | ~ 343,000 | 1860.61 |
| Hs746T | ~ 188,000 | 3540.54 |
| SNU-620 | ~ 124,000 | 2298.88 |
| MKN-45 | ~ 94,000 | 2523.17 |
| SNU-1 (cMet expressing negative control) | 0 | 84.45 |

| Colorectal cancer | | |
|---|---|---|
| HT-29 | ~ 24,000 | 227.74 |
| NCI-H747 | ~ 22,000 | 257.43 |
| HCT-116 | ~ 10,000 | 239.87 |
| SW48 | ~ 100 | 3.55 |

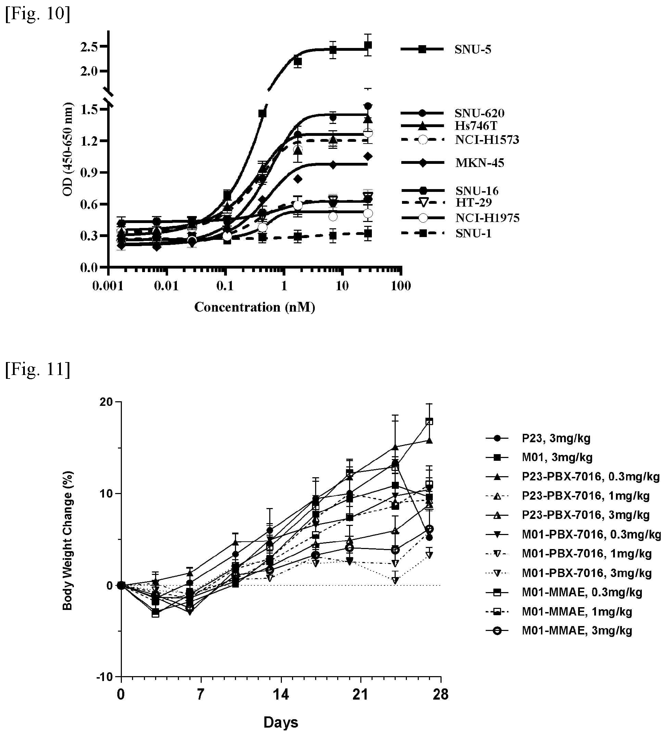

In summary, the M01 antibody bound to cell lines expressing the c-Met protein in a dose-dependent manner (Fig. 10), and the binding of the anti-c-Met ADC of the present invention to each cell line also showed the same pattern. That is, it was confirmed that even when the M01 antibody was conjugated to the payload via the linker, it did not significantly affect the binding affinity of the antibody.

### Experimental Example 3: Confirmation of Anticancer Efficacy of PBX Payload Anti-c-Met ADC

### Experimental Example 3-1. Confirmation of Anticancer Effect in Xenograft Model of Hs746T Human Gastric cancer

It was aimed to confirm the anticancer effect of the ADC of the present invention from the Hs746T human gastric cancer xenograft model of BALB/c nude mice (Zhejiang Vital River Laboratory Animal Technology Co., LTD.).

### Cell Culture

Hs746T tumor cells (ATCC, Catalog No. HTB-135) were cultured as a monolayer in DMEM medium supplemented with 10% FBS and 1% antibiotic-antimycotic at 37°C in an atmosphere of 5% CO₂ in air. Tumor cells (Hs746T) were routinely passaged three times a week by treating with trypsin-EDTA. The cells with exponential growth were harvested and counted for tumor inoculation.

### Inoculation of Tumor and Grouping of Animals

Mice were randomly assigned to groups of six each, and tumor cells were inoculated into the mice according to the experimental conditions shown in Table 9 below. Specifically, Hs746T tumor cells (2 × 10⁶ cells/mouse) were subcutaneously inoculated into the right flank of each mouse together with 0.1 mL of PBS containing Matrigel. The teatment was started on day 15 after inoculation, when the average tumor volume reached 197 mm³, and the date when the treatment started was set as Day 0. This experiment was performed for 27 days.

**[Table 9]**

| Group | Mouse Population | Treatment | Administration Dose (mg/kg) | Administration Dose per Volume (mL/kg) | Administration Route | Schedule |
|---|---|---|---|---|---|---|
| 1 | 6 | P23 (Isotype mAb) | 3 | 5 | i.v. | Single dose |
| 2 | 6 | M01(Anti-cMet mAb) | 3 | 5 | i.v. | Single dose |
| 3 | 6 | P23-PBX-7016 | 0.3 | 5 | i.v. | Single dose |
| 4 | 6 | P23-PBX-7016 | 1 | 5 | i.v. | Single dose |
| 5 | 6 | P23-PBX-7016 | 3 | 5 | i.v. | Single dose |
| 6 | 6 | M01-PBX-7016 (Example 3) | 0.3 | 5 | i.v. | Single dose |
| 7 | 6 | M01-PBX-7016 (Example 3) | 1 | 5 | i.v. | Single dose |
| 8 | 6 | M01-PBX-7016 (Example 3) | 3 | 5 | i.v. | Single dose |
| 9 | 6 | M01-MMAE | 0.3 | 5 | i.v. | Single dose |
| 10 | 6 | M01-MMAE | 1 | 5 | i.v. | Single dose |
| 11 | 6 | M01-MMAE | 3 | 5 | i.v. | Single dose |

### Inoculation of Tumor and Confirmation of Endpoints

The experimental endpoints were set when tumor growth stopped or the mice were cured. The size and volume of the tumor were measured twice a week in two and three dimensions using calipers, and these were used to calculate the T/C (tumor volume over control volume) and TGI (tumor growth inhibition) values.

In the T/C values (%) above, T and C represent the average volume of the experimental group and the control group on a specific day, respectively, and the T/C value is an index for the antitumor effect.

The TGI values were calculated using the following equation, where Tᵢ is a mean tumor volume of the treatment group on a specific date, T₀ is a mean tumor volume of the treatment group on the first day of treatment, Vᵢ is a mean tumor volume of the vehicle control group on the same day as Tᵢ, and V₀ is a mean tumor volume of the vehicle group on the first day of treatment. $TGI \left(%\right) = \left[1-\left({T}_{i}-{T}_{0}\right)/\left({V}_{i}-{V}_{0}\right)\right] \times 100$

### Statistics and Results Analysis

The mean and standard error of the mean (SEM) of tumor volume over time for each group are as shown in Tables 10 and 11 below.

On day 13 after the start of the experiment, the tumor volumes of individual mice in the isotype mAb control group began to exceed the sacrifice criterion of 3,000 mm³; therefore, statistical analysis was performed based on the results of the tumor volume measured on day 13. Since the data did not follow a normal distribution, the Kruskal-Wallis test was performed to compare tumor volumes between groups, followed by Dunn's *post hoc* analysis. All data were analyzed using SPSS 29.0. *p* < 0.05 was considered statistically significant.

**[Table 10]**

| Tumor Volume Over Time (Groups 1 to 5) | | | | | |
|---|---|---|---|---|---|
| Days | Tumor Volume (mm³) | | | | |
| | P23 (Isotype mAb) 3 mg/kg | M01 3 mg/kg | P23-PBX-7016 0.3 mg/kg | P23-PBX-7016 1 mg/kg | P23-PBX-7016 3 mg/kg |
| 0 | 197 ± 22 | 197 ± 24 | 197 ± 24 | 197 ± 24 | 197 ± 23 |
| 3 | 323 ± 73 | 291 ± 54 | 309 ± 37 | 298 ± 36 | 281 ± 55 |
| 6 | 523 ± 126 | 378 ± 80 | 512 ± 87 | 476 ± 80 | 318 ± 72 |
| 10 | 982 ± 268 | 562 ± 146 | 873 ± 170 | 807 ± 171 | 308 ± 80 |
| 13 | 1,575 ± 419 | 910 ± 233 | 1,205 ± 253 | 1,189 ± 243 | 405 ± 125 |
| 17 | 2,037 ± 382 (n=5) | 1,323 ± 287 | 1,714 ± 374 | 1,706 ± 325 | 669 ± 192 |
| 20 | 2,105 ± 72 (n=4) | 1,562 ± 342 | 2149 ± 475 | 2,089 ± 416 | 826 ± 223 |
| 24 | 3,016 ± 278 (n=4) | 2,141 ± 520 | 1,968 ± 619 (n = 4) | 1,918 ± 502 (n=4) | 1,140 ± 306 |
| 27 | 3336 (n=1) | 1,512 ± 394 (n=3) | 2,175 ± 874 (n = 3) | 1,873 ± 359 (n = 3) | 1,646 ± 416 |

**[Table 11]**

| Tumor Volume Over Time (Groups 6 to 11) | | | | | | |
|---|---|---|---|---|---|---|
| Days | Tumor Volume (mm³) | | | | | |
| | M01-PBX-7016 (Example 3) 0.3 mg/kg | M01-PBX-7016 (Example 3) 1 mg/kg | M01-PBX-7016 (Example 3) 3 mg/kg | M01-MMAE 0.3 mg/kg | M01-MMAE 1 mg/kg | M01-MMAE 3 mg/kg |
| 0 | 197 ± 22 | 197 ± 25 | 197 ± 23 | 197 ± 22 | 197 ± 24 | 197 ± 25 |
| 3 | 281 ± 30 | 300 ± 64 | 286 ± 50 | 310 ± 65 | 307 ± 25 | 253 ± 52 |
| 6 | 357 ± 40 | 334 ± 89 | 234 ± 31 | 459 ± 68 | 387 ± 27 | 221 ± 75 |
| 10 | 455 ± 74 | 279 ± 110 | 149 ± 27 | 713 ± 129 | 534 ± 35 | 193 ± 97 |
| 13 | 677 ± 125 | 271 ± 137 | 115 ± 23 | 1,034 ± 132 | 737 ± 48 | 221 ± 128 |
| 17 | 1,092 ± 227 | 310 ± 233 | 65 ± 13 | 1,705 ± 146 | 1,015 ± 59 | 356 ± 199 |
| 20 | 1,335 ± 284 | 362 ± 317 | 31 ± 5 | 2,169 ± 283 | 1,188 ± 107 | 434 ± 234 |
| 24 | 1,851 ± 365 | 506 ± 455 | 23 ± 4 | 2542 ± 256 (n=5) | 1,477 ± 124 | 630 ± 318 |
| 27 | 1,648 ± 122 (n=4) | 810 ± 718 | 15 ± 2 | 3,230 ± 346 (n=4) | 2,120 ± 192 | 826 ± 385 |

### Mortality, Morbidity, and Weight Gain/Loss

The body weight of mice was monitored regularly as an indirect measure of toxicity. No significant weight loss was observed during the experimental period. Body weight changes in each treatment group are shown in Fig. 11.

### Tumor Volume

The mean tumor volume over time in the experimental mice is shown in Tables 10 and 11 above.

### Tumor Growth Inhibition Assay and Tumor Growth Curve

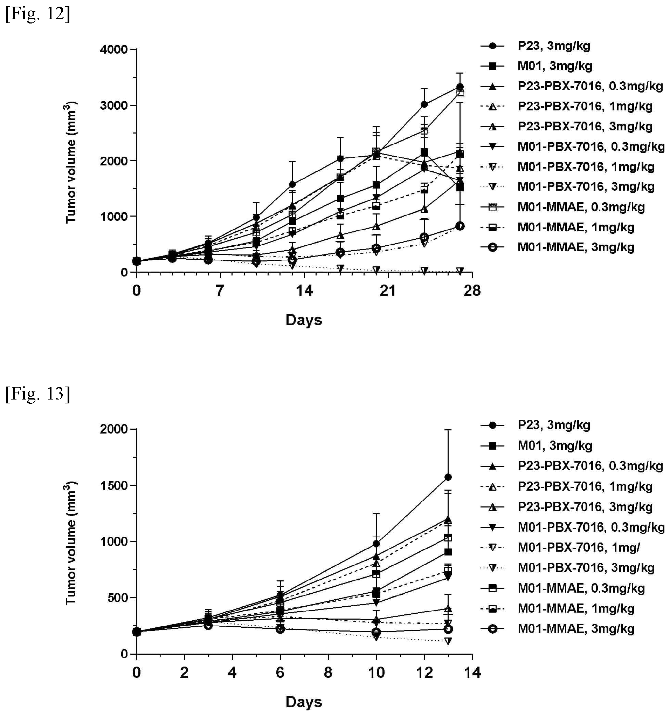

Tumor growth inhibition assays in experimental mice were performed 13 days after the start of the experiment and calculated based on tumor volume measurements. The results are shown in Table 12. Tumor growth curves are shown in Figs. 12 and 13.

**[Table 12]**

| Treatment | Population | Tumor volume (mm³) 13 Days | T/C (%) | TGI (%) | *p* Value |
|---|---|---|---|---|---|
| P23 (Isotype mAb), 3 mg/kg | 6 | 1,575 ± 419 | - | - | - |
| M01, 3 mg/kg | 6 | 910 ± 233 | 57.75 | 48.28 | >0.999 |
| P23-PBX-7016, 0.3 mg/kg | 6 | 1,205± 253 | 76.49 | 26.87 | >0.999 |
| P23-PBX-7016, 1 mg/kg | 6 | 1,189 ± 243 | 75.52 | 27.98 | >0.999 |
| P23-PBX-7016, 3 mg/kg | 6 | 405 ± 125 | 25.71 | 84.88 | 0.209 |
| M01-PBX-7016 (Example 3), 0.3 mg/kg | 6 | 677 ± 125 | 42.97 | 65.17 | >0.999 |
| M01-PBX-7016 (Example 3), 1 mg/kg | 6 | 271 ± 137 | 17.22 | 94.59 | 0.052 |
| M01-PBX-7016 (Example 3), 3 mg/kg | 6 | 115 ± 23 | 7.28 | 105.95 | 0.003 |
| M01-MMAE, 0.3 mg/kg | 6 | 1,034 ± 132 | 65.67 | 39.22 | >0.999 |
| M01-MMAE, 1 mg/kg | 6 | 737 ± 48 | 46.78 | 60.83 | >0.999 |
| M01-MMAE, 3 mg/kg | 6 | 221 ± 128 | 14.04 | 98.23 | 0.009 |

### Results

In order to confirm the anticancer effect of the ADC of the present invention in a subcutaneous xenograft mouse model with Hs746T human gastric cancer, mouse mortality, body weight increase/decrease rate, tumor volume, tumor growth inhibition rate, tumor weight, and tumor growth curve were analyzed.

The mean tumor volume in the group administered with P23 (isotype mAb) reached 1,575 mm³ on day 13 after the start of administration. Compared with the isotype mAb group, P23-PBX-7016 (3 mg/kg) showed the value of T/C = 25.71%, TGI = 84.88%, *p* = 0.209, M01-PBX-7016 (1 mg/kg) showed the value of T/C = 17.22%, TGI = 94.59%, *p* = 0.052, M01-PBX-7016 (3 mg/kg) showed the value of T/C = 7.28%, TGI = 105.95%, *p* = 0.003, and M01-MMAE (3 mg/kg) showed the value of T/C = 14.04%, TGI = 98.23%, *p* = 0.009, and thus, in the treated groups, the antitumor effect was observed with the mean tumor volumes being 405mm³, 271 mm³, 115 mm³, and 221 mm³ on day 13 after the start of treatment. Therefore, it can be seen that the antibody-drug conjugate (ADC) of the present invention exhibits excellent tumor growth inhibitory activity. Meanwhile, no significant weight loss was observed during the experiment.

### Experimental Example 3-2. Confirmation of Anticancer Effect in LU-01-0439 Human Lung Cancer Xenograft Model

It was aimed to confirm the anticancer effect of the ADC of the present invention from the LU-01-0439 human lung cancer xenograft model of BALB/c nude mice (Zhejiang Vital River Laboratory Animal Technology Co., LTD.).

### Creating PDX model

The lung cancer PDX model of LU-01-0439 was generated by transplanting surgically resected clinical samples into immunodeficient mice and was established as passage 0 (P0). Then, the cells were subcultured through serial transplantation, and the passage after P0 was defined as passage 1 (P1). The tumor recovered from the frozen P2 tumor tissue was designated as FP3, and finally, FP4 tumor tissue was prepared for the experiment.

The H-score assessed by immunohistochemical staining with SP44 (Ventana), an anti-c-Met antibody of LU-01-0439, was evaluated as 179 points according to the criteria in Table 13 below.

**[Table 13]**

| | |
|---|---|
| 0 | No staining |
| 1+ | Weak intensity (clearly visible at 200x magnification) |
| 2+ | Medium intensity (clearly visible at 100x magnification) |
| 3+ | Strong intensity (clearly visible at 40x magnification) |

### Tumor Transplantation and Grouping of Animals

Mice were randomly assigned to groups of eight each, and tumors were implanted into the mice according to the experimental conditions shown in Table 14 below. Specifically, LU-01-0439 FP4 tumor fragments (20-30 mm³) were subcutaneously implanted into the right flank of each mouse. Treatment was started on day 24 after tumor transplantation, when the mean tumor volume reached 175 mm³, and the treatment start date was set as Day 0. This experiment was performed for 35 days.

**[Table 14]**

| Group | Mouse Population | Treatment | Administration Dose (mg/kg) | Admintration volume (mL/kg) | Administration Route | Schedule |
|---|---|---|---|---|---|---|
| 1 | 8 | P23 (Isotype mAb) | 10 | 5 | i.v. | QW × 2 weeks |
| 2 | 8 | M01 (Anti-cMet mAb) | 10 | 5 | i.v. | QW × 2 weeks |
| 3 | 8 | P23-PBX-7016 | 10 | 5 | i.v. | QW × 2 weeks |
| 4 | 8 | M01-MMAE | 1 | 5 | i.v. | QW × 2 weeks |
| 5 | 8 | M01-MMAE | 3 | 5 | i.v. | QW × 2 weeks |
| 6 | 8 | M01-MMAE | 10 | 5 | i.v. | QW × 2 weeks |
| 7 | 8 | M01-PBX-7016 (Example 3) | 1 | 5 | i.v. | QW × 2 weeks |
| 8 | 8 | M01-PBX-7016 (Example 3) | 3 | 5 | i.v. | QW × 2 weeks |
| 9 | 8 | M01-PBX-7016 (Example 3) | 10 | 5 | i.v. | QW × 2 weeks |

### Tumor Measurement and Confirmation of Endpoints

The tumor size was measured using the same method as in Experimental Example 3-1 above.

### Analysis of Statistics and Results

The mean and standard error of the mean (SEM) of tumor volume over time for each group are as shown in Tables 15 and 16 below.

Statistical analysis was performed on the data obtained 35 days after the start of the experiment to determine differences in tumor volume and tumor weight between groups. Since the data did not follow a normal distribution, the Kruskal-Wallis test was performed to compare tumor volumes between groups, followed by Dunn's *post hoc* analysis. All data were analyzed using SPSS 29.0. *p* < 0.05 was considered statistically significant.

**[Table 15]**

| Tumor Volume Over Time (Groups 1 to 6) | | | | | | |
|---|---|---|---|---|---|---|
| Days | Tumor Volume (mm³) | | | | | |
| | P23 (Isotype mAb) 10 mg/kg | M01 10 mg/kg | P23-PBX-7016 10 mg/kg | M01-MMAE 1 mg/kg | M01-MMAE 3 mg/kg | M01-MMAE 10 mg/kg |
| 0 | 175 ± 15 | 175 ± 16 | 175 ± 15 | 175 ± 15 | 175 ± 15 | 175 ± 15 |
| 3 | 219 ± 21 | 228 ± 25 | 209 ± 22 | 217 ± 25 | 201 ± 22 | 208 ± 24 |
| 7 | 281 ± 30 | 299 ± 34 | 238 ± 22 | 273 ± 36 | 240 ± 35 | 237 ± 30 |
| 10 | 343 ± 42 | 407 ± 45 | 247 ± 19 | 399 ± 58 | 297 ± 41 | 284 ± 35 |
| 14 | 455 ± 58 | 538 ± 55 | 217 ± 18 | 572 ± 92 | 401 ± 64 | 403 ± 58 |
| 17 | 568 ± 74 | 738 ± 82 | 217 ± 28 | 710 ± 120 | 487 ± 82 | 543 ± 87 |
| 21 | 736 ± 113 | 894 ± 106 | 205 ± 42 | 830 ± 139 | 556 ± 92 | 635 ± 126 |
| 24 | 874 ± 135 | 1,168 ± 134 | 263 ± 61 | 973 ± 148 | 690 ± 95 | 805 ± 160 |
| 28 | 1,098 ± 188 | 1,425 ± 167 | 393 ± 90 | 1,258 ± 202 | 852 ± 106 | 966 ± 159 |
| 31 | 1,344 ± 228 | 1,690 ± 214 | 536 ± 117 | 1,523 ± 228 | 1,027 ± 113 (n=7) | 1,169 ± 175 |
| 35 | 1,460 ± 240 | 1,655 ± 245 | 630 ± 137 | 1,650 ± 239 | 1,141 ± 138 (n=7) | 1,378 ± 227 |

**[Table 16]**

| Tumor Volume Over Time (Groups 7 to 9) | | | |
|---|---|---|---|
| Days | Tumor Volume (mm³) | | |
| | M01-PBX-7016 (Example 3) 1 mg/kg | M01-PBX-7016 (Example 3) 3 mg/kg | M01-PBX-7016 (Example 3) 10 mg/kg |
| 0 | 175 ± 15 | 175 ± 15 | 175 ± 16 |
| 3 | 222 ± 22 | 234 ± 28 | 231 ± 35 |
| 7 | 231 ± 26 | 223 ± 28 | 212 ± 28 |
| 10 | 218 ± 28 | 174 ± 25 | 171 ± 23 |
| 14 | 202 ± 24 | 114 ± 21 | 112 ± 21 |
| 17 | 192 ± 24 | 90 ± 20 | 84 ± 14 |
| 21 | 152 ± 27 | 42 ± 8 | 42 ± 11 |
| 24 | 170 ± 36 | 34 ± 6 | 33 ± 9 |
| 28 | 256 ± 60 | 26 ± 5 | 23 ± 6 |
| 31 | 362 ± 95 | 18 ± 4 | 15 ± 5 |
| 35 | 394 ± 89 | 7 ± 4 | 7 ± 3 |

### Mortality, Morbidity, and Weight Gain/Loss

At 28 days after the start of the experiment, one mouse in Group 5 (M01-MMAE, 3 mg/kg) died due to severe weight loss. The autopsy results showed that the liver and kidneys were pale and there were numerous subcutaneous hemorrhages. However, since no such results were observed in other mice, the incidence was low, and the response according to the treatment dose could not be confirmed, it was unclear whether the administration of the experimental substance affected the death.

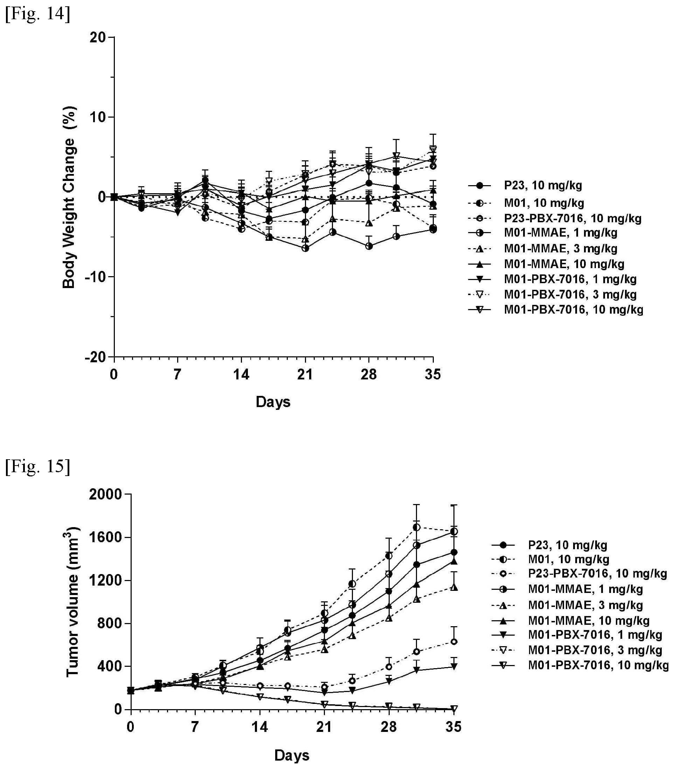

The body weight of mice was monitored regularly as an indirect measure of toxicity. No significant weight loss was observed during the experimental period except for the above-mentioned mouse. The body weight change in each treatment group is as shown in Fig. 14.

### Tumor Volume

The mean tumor volume over time in the experimental mice is as shown in Tables 15 and 16 above.

### Tumor Growth Inhibition Assay

Tumor growth inhibition assays in experimental mice were performed 35 days after the start of the experiment and calculated based on tumor volume measurements. The results are as shown in Table 17.

**[Table 17]**

| Treatment | Populati on | Tumor Volume (mm³) 35 Days | T/C (%) | TGI (%) | *p* Value |
|---|---|---|---|---|---|
| P23 (Isotype mAb), 10 mg/kg | 8 | 1,460 ± 240 | - | - | - |
| M01, 10 mg/kg | 8 | 1,655 ± 245 | 113.37 | -15.18 | >0.999 |
| P23-PBX-7016, 10 mg/kg | 8 | 630 ± 137 | 43.11 | 64.62 | >0.999 |
| M01-MMAE, 1 mg/kg | 8 | 1,650 ± 239 | 113.00 | -14.77 | >0.999 |
| M01-MMAE, 3 mg/kg | 7 | 1,141 ± 138 | 78.11 | 24.86 | >0.999 |
| M01-MMAE, 10 mg/kg | 8 | 1,378 ± 227 | 94.36 | 6.40 | >0.999 |
| M01-PBX-7016 (Example 3), 1 mg/kg | 8 | 394 ± 89 | 27.01 | 82.92 | 0.307 |
| M01-PBX-7016 (Example 3), 3 mg/kg | 8 | 7 ± 4 | 0.45 | 113.08 | 0.001 |
| M01-PBX-7016 (Example 3), 10 mg/kg | 8 | 7 ± 3 | 0.45 | 113.09 | 0.003 |

### Tumor Weight

Assays of tumor weight of experimental mice were performed 35 days after the start of the experiment. The results are as shown in Table 18.

**[Table 18]**

| Treatment | Populati on | Tumor Weight (g) on Day 35 | T/C (%) | *p* Value |
|---|---|---|---|---|
| P23 (Isotype mAb), 10 mg/kg | 8 | 1.307 ± 0.237 | - | - |
| M01, 10 mg/kg | 8 | 1.307 ± 0.204 | 100.00 | >0.999 |
| P23-PBX-7016, 10 mg/kg | 8 | 0.561 ± 0.117 | 42.93 | >0.999 |
| M01-MMAE, 1 mg/kg | 8 | 1.628 ± 0.270 | 124.52 | >0.999 |
| M01-MMAE, 3 mg/kg | 7 | 1.171 ± 0.160 | 89.55 | >0.999 |
| M01-MMAE, 10 mg/kg | 8 | 1.375 ± 0.247 | 105.13 | >0.999 |
| M01-PBX-7016 (Example 3), 1 mg/kg | 8 | 0.417 ± 0.095 | 31.89 | 0.859 |
| M01-PBX-7016 (Example 3), 3 mg/kg | 8 | 0.007 ± 0.003 | 0.54 | 0.002 |
| M01-PBX-7016 (Example 3), 10 mg/kg | 8 | 0.009 ± 0.002 | 0.72 | 0.006 |

### Tumor growth curve

The tumor growth curves in each group are as shown in Fig. 15.

### Results

In order to confirm the anticancer effect of the ADC of the present invention in LU-01-0439, which is a human lung cancer subcutaneous xenograft mouse model, mouse mortality rate, body weight increase/decrease rate, tumor volume, tumor growth inhibition rate, tumor weight, and tumor growth curve were analyzed.

Except for one mouse in Group 5 (M01-MMAE, 3 mg/kg) that showed a significant weight loss on day 28 from the start of the experiment during the experimental period, no significant weight loss was observed in the other mice. Even in the mice where the weight loss was observed, no significant correlation was observed between the administered substance and the weight loss.

The mean tumor volume of the group, where P23 (isotype mAb) was administered, reached 1,460 mm³ on day 35 after the start of administration. Compared to the isotype mAb group, in the groups where M01-PBX-7016 (Example 3) was administered at 3 mg/kg and 10 mg/kg (T/C = 0.45% and 0.45%, TGI = 113.08% and 113.09%, *p* = 0.001 and 0.003), on day 35 after the start of administration, the mean tumor volume was reduced to 7 mm³ and 7 mm³, respectively, thus confirming statistically significant antitumor activity. Therefore, it can be seen that the antibody-drug conjugate (ADC) of the present invention exhibits excellent tumor growth inhibitory activity.

### Experimental Example 3-3. Confirmation of Anticancer Effect in Xenograft Model of NCI-H441 Human Lung Cancer

It was aimed to confirm the anticancer effect of the ADC of the present invention from the NCI-H441 human lung cancer xenograft model of BALB/c nude mice (Zhejiang Vital River Laboratory Animal Technology Co., LTD.).

### Cell Culture

NCI-H441 tumor cells (ATCC, Catalog No. HTB-174) were maintained *in vitro* in RPMI 1640 medium supplemented with 10% FBS and 1% antibiotic-antimycotic at 37°C and 5% CO₂ in air. Tumor cells NCI-H441 were routinely passaged twice a week by treatment with trypsin-EDTA. The cells with exponential growth were harvested and counted for tumor inoculation.

### Tumor Inoculation and Grouping of Animals

Mice were randomly assigned to groups of 10 each, and tumor cells were inoculated into the mice according to the experimental conditions shown in Table 19 below. Specifically, NCI-H441 tumor cells (5 × 10⁶ cells/mouse) were subcutaneously inoculated into the right flank of each mouse together with 0.2 mL of PBS containing Matrigel (1:1). Treatment was started on day 14 after inoculation, when the mean tumor volume reached 180 mm³, and the treatment start date was set as day 0. This experiment was performed for 35 days.

**[Table 19]**

| Group | Mouse Population (N) | Treatment | Administrati on Dose (mg/kg) | Administra tion Volume (mg/mL) | Administ ration Route | Schedule |
|---|---|---|---|---|---|---|
| 1 | 10 | P23 (Isotype mAb) | 3 | 5 | i.v. | QW × 2 weeks |
| 2 | 10 | M01 (Anti-cMet mAb) | 3 | 5 | i.v. | QW × 2 weeks |
| 3 | 10 | P23-MMAE | 3 | 5 | i.v. | QW × 2 weeks |
| 4 | 10 | P23-PBX-7016 | 3 | 5 | i.v. | QW × 2 weeks |
| 5 | 10 | M01-MMAE | 0.3 | 5 | i.v. | QW × 2 weeks |
| 6 | 10 | M01-MMAE | 1 | 5 | i.v. | QW × 2 weeks |
| 7 | 10 | M01-MMAE | 3 | 5 | i.v. | QW × 2 weeks |
| 8 | 10 | M01-PBX-7016 (Example 3) | 0.3 | 5 | i.v. | QW × 2 weeks |
| 9 | 10 | M01-PBX-7016 (Example 3) | 1 | 5 | i.v. | QW × 2 weeks |
| 10 | 10 | M01-PBX-7016 (Example 3) | 3 | 5 | i.v. | QW × 2 weeks |

### Tumor Measurement and Confirmation of Endpoints

The tumor size was measured using the same method as in Experimental Example 3-1 above.

### Analysis of Statistics and Results

The mean and standard error of the mean (SEM) of tumor volume over time for each group are as shown in Tables 20 and 21 below.

Statistical analysis was performed on the data obtained 35 days after the start of the experiment to determine differences in tumor volume and tumor weight between groups. Since the data did not follow a normal distribution, the Kruskal-Wallis test was performed to compare tumor volumes between groups, followed by Dunn's *post hoc* analysis. All data were analyzed using SPSS 29.0. *p* < 0.05 was considered statistically significant.

**[Table 20]**

| Tumor Volume Over Time (Groups 1 to 5) | | | | | |
|---|---|---|---|---|---|
| Days | Tumor Volume (mm³) | | | | |
| | P23 (Isotype mAb) 3 mg/kg | M01 3 mg/kg | P23-MMAE 3 mg/kg | P23-PBX-7016 3 mg/kg | M01-MMAE 0.3 mg/kg |
| 0 | 180 ± 13 | 180 ± 13 | 180 ± 13 | 180 ± 13 | 180 ± 12 |
| 4 | 204 ± 17 | 199 ± 13 | 208 ± 19 | 198 ± 16 | 212 ± 17 |
| 7 | 246 ± 22 | 234 ± 16 | 244 ± 26 | 171 ± 14 | 229 ± 18 |
| 11 | 293 ± 26 | 254 ± 20 | 283 ± 36 | 140 ± 14 | 264 ± 22 |
| 14 | 335 ± 31 | 273 ± 25 | 287 ± 39 | 131 ± 14 | 285 ± 22 |
| 18 | 506 ± 49 | 348 ± 36 | 358 ± 66 | 134 ± 15 | 373 ± 40 |
| 21 | 639 ± 59 | 467 ± 47 | 396 ± 71 | 138 ± 17 | 434 ± 53 |
| 25 | 826 ± 74 | 734 ± 86 | 563 ± 93 | 155 ± 20 | 584 ± 77 |
| 28 | 983 ± 84 | 882 ± 108 | 708 ± 119 | 190 ± 33 | 751 ± 96 |
| 32 | 1,286 ± 115 | 1,151 ± 156 | 977 ± 193 | 235 ± 42 | 973 ± 130 |
| 35 | 1,593 ± 140 | 1,431 ± 157 | 1,222 ± 218 | 303 ± 54 | 1,301 ± 172 |

**[Table 21]**

| Tumor Volume Over Time (Groups 6 to 10) | | | | | |
|---|---|---|---|---|---|
| Days | Tumor Volume (mm³) | | | | |
| | M01-MMAE | | M01-PBX-7016 (Example 3) | | |
| | 1 mg/kg | 3 mg/kg | 0.3 mg/kg | 1 mg/kg | 3 mg/kg |
| 0 | 180 ± 13 | 180 ± 13 | 180 ± 13 | 180 ± 13 | 180 ± 13 |
| 4 | 189 ± 15 | 184 ± 18 | 184 ± 16 | 191 ± 15 | 192 ± 20 |
| 7 | 164 ± 17 | 176 ± 26 | 140 ± 13 | 126 ± 14 | 128 ± 16 |
| 11 | 146 ± 16 | 147 ± 25 | 121 ± 12 | 101 ± 13 | 104 ± 14 |
| 14 | 136 ± 16 | 121 ± 21 | 106 ± 11 | 88 ± 9 | 85 ± 13 |
| 18 | 118 ± 15 | 100 ± 19 | 78 ± 7 | 68 ± 7 | 68 ± 10 |
| 21 | 100 ± 13 | 79 ± 15 | 68 ± 6 | 54 ± 5 | 53 ± 8 |
| 25 | 121 ± 19 | 66 ± 12 | 64 ± 6 | 48 ± 4 | 49 ± 7 |
| 28 | 162 ± 26 | 57 ± 11 | 56 ± 7 | 36 ± 2 | 41 ± 7 |
| 32 | 223 ± 40 | 46 ± 9 | 53 ± 10 | 30 ± 2 | 37 ± 7 |
| 35 | 305 ± 59 | 42 ± 8 | 55 ± 15 | 18 ± 1 | 28 ± 12 |

### Mortality, Morbidity, and Weight Gain/Loss

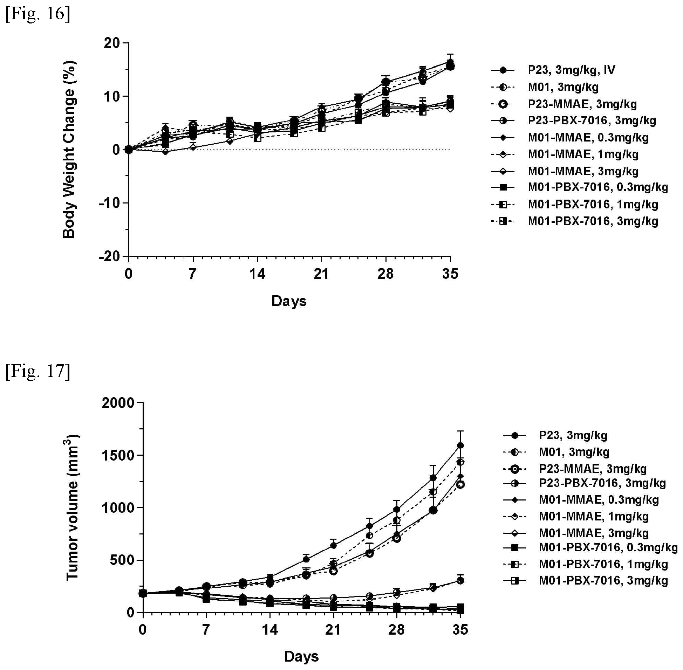

The body weight of mice was monitored regularly as an indirect measure of drug toxicity. No significant body weight loss was observed during the experimental period, and the body weight change in each treatment group is as shown in Fig. 16. No disease onset or death occurred.

### Tumor volume

The mean tumor volume over time in the experimental mice is as shown in Tables 20 and 21 above.

### Tumor growth inhibition assay

Tumor growth inhibition assays in experimental mice were performed 35 days after the start of the experiment and calculated based on tumor volume measurements. The results are as shown in Table 22.

**[Table 22]**

| Treatment | Populati on | Tumor Volume (mm³) on Day 35 | T/C (%) | TGI (%) | *p* Value |
|---|---|---|---|---|---|
| P23 (Isotype mAb), 3 mg/kg | 10 | 1,593 ± 140 | - | - | - |
| M01, 3 mg/kg | 10 | 1,431 ± 157 | 89.85 | 11.44 | >0.999 |
| P23-MMAE, 3 mg/kg | 10 | 1,222 ± 218 | 76.70 | 26.27 | >0.999 |
| P23-PBX-7016, 3 mg/kg | 10 | 303 ± 54 | 19.00 | 91.34 | 0.203 |
| M01-MMAE, 0.3 mg/kg | 10 | 1,301 ± 172 | 81.67 | 20.67 | >0.999 |
| M01-MMAE, 1 mg/kg | 10 | 305 ± 59 | 19.14 | 91.18 | 0.286 |
| M01-MMAE, 3 mg/kg | 10 | 42 ± 8 | 2.61 | 109.83 | <0.001 |
| M01-PBX-7016 (Example 3), 0.3 mg/kg | 10 | 55 ± 15 | 3.48 | 108.84 | 0.002 |
| M01-PBX-7016 (Example 3), 1 mg/kg | 10 | 18 ± 1 | 1.15 | 111.47 | <0.001 |
| M01-PBX-7016 (Example 3), 3 mg/kg | 10 | 28 ± 12 | 1.76 | 110.78 | <0.001 |

### Tumor Weight

The analysis of tumor weight of experimental mice was performed 35 days after the start of the experiment. The results are as shown in Table 23.

**[Table 23]**

| Treatment | Population | Tumor Weight (g) on Day 35 | T/C_{weight} (%) | *p* Value |
|---|---|---|---|---|
| P23(Isotype mAb), 3 mg/kg | 10 | 1.670 ± 0.158 | - | - |
| M01, 3 mg/kg | 10 | 1.510 ± 0.145 | 90.47 | >0.999 |
| P23-MMAE, 3 mg/kg | 10 | 1.342 ± 0.247 | 80.39 | >0.999 |
| P23-PBX-7016, 3 mg/kg | 10 | 0.318 ± 0.059 | 19.05 | 0.177 |
| M01-MMAE, 0.3 mg/kg | 10 | 1.368 ± 0.186 | 81.95 | >0.999 |
| M01-MMAE, 1 mg/kg | 10 | 0.343 ± 0.067 | 20.57 | 0.395 |
| M01-MMAE, 3 mg/kg | 10 | 0.048 ± 0.012 | 2.86 | <0.001 |
| M01-PBX-7016 (Example 3), 0.3 mg/kg | 10 | 0.058 ± 0.014 | 3.48 | 0.002 |
| M01-PBX-7016 (Example 3), 1 mg/kg | 10 | 0.019 ± 0.001 | 1.14 | <0.001 |
| M01-PBX-7016 (Example 3), 3 mg/kg | 10 | 0.033 ± 0.013 | 1.99 | <0.001 |

### Tumor growth curves

The tumor growth curves in each group are as shown in Fig. 17.

### Results

In order to confirm the anticancer effect of the ADC of the present invention in the NCI-H441 human lung cancer subcutaneous xenograft mouse model, mouse mortality, body weight increase/decrease rate, tumor volume, tumor growth inhibition rate, tumor weight, and tumor growth curves were analyzed.

The mean tumor volume of the group, where P23 (isotype mAb) was administered, reached 1,593 mm³ on day 35 after the start of administration. Compared to the isotype mAb group, in the groups where M01-MMAE (T/C = 2.61%, TGI = 109.83%, p<0.001) was administered at 3 mg/kg, and where M01-PBX-7016 was administered at 0.3 mg/kg (T/C = 3.48%, TGI = 108.84%, *p* = 0.002), 1 mg/kg (T/C = 1.15%, TGI = 111.47%, *p*<0.001), and 3 mg/kg (T/C = 1.76%, TGI = 110.78%, *p*<0.001), on day 35 after the start of administration, the mean tumor volume was reduced to 42 mm³, 55 mm³, 18 mm³, and 28 mm³, respectively, thus confirming statistically significant antitumor activity. Therefore, it can be seen that the antibody-drug conjugate (ADC) of the present invention exhibits excellent tumor growth inhibitory activity.

### Sequence Listing

The sequence listing of the antibodies used in the present invention is as described in Table 24 below.

**[Table 24]**

| SEQ ID NO: | Name of Sequence | Sequence |
|---|---|---|
| 1 | M01 (anti-c-Met antibody) CDR-H1 | GYIFTAYT |
| 2 | M01 (anti-c-Met antibody) CDR-H2 | IKPNNGLA |
| 3 | M01 (anti-c-Met antibody) CDR-H3 | ARSEITTEFDY |
| 4 | M01 (anti-c-Met antibody) CDR-L1 | ESVDSYANSF |
| 5 | M01 (anti-c-Met antibody) CDR-L2 | RAS |
| 6 | M01 (anti-c-Met antibody) CDR-L3 | QQSKEDPLT |
| 7 | M01 (anti-c-Met antibody) heavy chain variable region | QVQLVQSGAEVKKPGASVKVSCKASGYIFTAYTMHWVRQ APGQGLEWMGWIKPNNGLANYAQKFQGRVTMTRDTSIST AYMELSRLRSDDTAVYYCARSEITTEFDYWGQGTLVTVSS |
| 8 | M01(anti-c-Met antibody) light chain variable region | DIVMTQSPDSLAVSLGERATINCKSSESVDSYANSFLHWYQ QKPGQPPKLLIYRASTRESGVPDRFSGSGSGTDFTLTISSLQA EDVAVYYCQQSKEDPLTFGGGTKVEIK |
| 9 | M01 (anti-c-Met antibody) heavy chain | QVQLVQSGAEVKKPGASVKVSCKASGYIFTAYTMHWVRQ APGQGLEWMGWIKPNNGLANYAQKFQGRVTMTRDTSIST AYMELSRLRSDDTAVYYCARSEITTEFDYWGQGTLVTVSS ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKKVEPKSCDKTHTCPPCPAPELLGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALPAPIEKTISKAKGQPREPQVYTLPPSRDELTKNQ VSLTCLVKGFYPSDIAVEWESNGQPENNYKTTPPVLDSDGS FFLYSKLTVDKSRWQQGNVFSCSVMHEALHNHYTQKSLSL SPGK |
| 10 | M01 (anti-c-Met antibody) light chain | DIVMTQSPDSLAVSLGERATINCKSSESVDSYANSFLHWYQ QKPGQPPKLLIYRASTRESGVPDRFSGSGSGTDFTLTISSLQA EDVAVYYCQQSKEDPLTFGGGTKVEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSS PVTKSFNRGEC |
| 11 | P63 (SARS-CoV-2 mAb) heavy chain | EVQLVQSGGGLIQPGGSLRLSCAASEFIVSSNYMTWVRQAP GKGLEWVSIIYPGGSTYYADSVKGRFTISRDNSKNTLYLRM NSLRAEDTAVYYCARDLPLTGTTLDYWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKRVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSRDELTKNQVSLT CLVKGFYPSDIAVEWESNGQPENNYKTTPPVLDSDGSFFLY SKLTVDKSRWQQGNVFSCSVMHEALHNHYTQKSLSLSPGK |
| 12 | P63 (SARS-CoV-2 mAb) light chain | ELVLTQSPSSLSASVGDRVTITCRASQSISSYLNWYQQKPGK APKLLIYAASSLQSGVPSRFSGSGSGTDFTLTISSLQPEDFAT YYCQQSYSTPLTFGGGTKLEIKRTVAAPSVFIFPPSDEQLKS GTASVVCLLNNFYPREAKVQWKVDNALQSGNSQES VTEQDSKDSTYSLSSTLTLSKADYEKHKVYACEVTHQGLSS PVTKSFNRGEC |
| 13 | Navivumab (antiinfluenza A mAb) heavy chain | MDWTWSILFLVAAATGAHSQVQLVQSGAEVKKPGASVKV SCKTSGYSFSTYGVSWVRQAPGQGPEWVGWISAYTGITDY AQKFQGRVTLTTDATTATAFLDLRSLRPDDTATYFCARDK VQGRVEVGSGGRHDYWGQGTLVIVSSASTKGPSVFPLAPSS KSTSGGTAALGCLVKDYFPEPVTVSWNSGALTSGVHTFPA VLQSSGLYSLSSVVTVPSSSLGTQTYICNVNHKPSNTKVDK KVEPKSCDKTHTCPPCPAPELLGGPSVFLFPPKPKDTLMISR TPEVTCVVVDVSHEDPEVKFNWYVDGVEVHNAKTKPREE QYNSTYRVVSVLTVLHQDWLNGKEYKCKVSNKALPAPIEK TISKAKGQPREPQVYTLPPSRDELTKNQVSLTCLVKGFYPSD IAVEWESNGQPENNYKTTPPVLDSDGSFFLYSKLTVDKSRW QQGNVFSCSVMHEGLHNHYTQKSLSLSPGK |
| 14 | Navivumab (antiinfluenza A mAb) light chain | METPAQLLFLLLLWLPDTTGEVVLTQSPGTLALPPGERATL SCRASHRVGSTYIAWYQQKSGQAPRRLIYGASNRATDIPDR FSGSGSGTDFTLTIRRLEPEDSAVYYCQQFSVSPWTFGQGTR VEIKRTVAAPSVFIFPPSDEQLKSGTASVVCLLNNFYPREAK VQWKVDNALQSGNSQESVTEQDSKDSTYSLSSTLTLSKAD YEKHKVYACEVTHQGLSSPVTKSFNRGEC |

## Claims

1. An antibody-drug conjugate (ADC) having a structure, in which a compound according to Formula 1 below or a pharmaceutically acceptable salt, a stereoisomer, a tautomer, a prodrug, a hydrate, a solvate, or an isotopically labeled analog thereof, is linked to an anti-c-Met antibody via a linker: 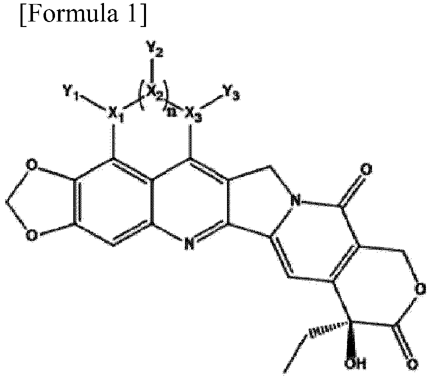 wherein:
X₁ and X₃ are each independently carbon, oxygen, nitrogen, or sulfur, and X₁ and X₃ are the same or different;
X₂ is carbon, oxygen, nitrogen, sulfur, a single bond, or a double bond;
n is 0, 1, or 2; and
Y₁, Y₂, and Y₃ are each independently hydrogen or a functional group comprising oxygen, nitrogen, phosphorus, or sulfur.

2. The antibody-drug conjugate of claim 1, wherein:
X_{1,} X₂, and X₃ are each independently carbon;
n is 1;
Y₁ and Y₂ are each independently hydrogen;
Y₃ is -NZ₁Z₂; and
Z₁ and Z₂ each comprise a functional group selected from the group consisting of hydrogen, halogen, hydroxyl, C₁₋₃ alkyl, C(=O)H, C(=O)(C₁₋₃ alkyl), C(=O)(C₂₋₃ alkenyl), C(=O)NH₂, C(=O)NH(C₁₋₃ alkyl), (CH₂)₂₋₄OH, (CH₂)₂₋₄O(C₁₋₃ alkyl), (CH₂)₂₋₄NH₂, (CH₂)₂₋₄NH(C₁₋₃ alkyl), (CH₂)₂₋₄N(C₁₋₃ alkyl)₂, and C(=O)CR¹R²R³, wherein R¹, R², and R³ are each independently hydrogen, hydroxyl, C₁₋₃ alkyl, C₃₋₇ cycloalkyl, or C₁₋₃ haloalkyl.

3. The antibody-drug conjugate of claim 1, wherein:
X_{1,} X₂, and X₃ are each independently carbon;
n is 1;
Y₁ and Y₂ are each independently hydrogen;
Y₃ is -NZ₁Z₂; and
Z₁ is hydrogen, and Z₂ is C(=O)(C₂₋₃ alkenyl) or C(=O)CR¹R²R³, wherein R¹, R², and R³ are each independently hydrogen, hydroxyl, C₁₋₃ alkyl, C₃₋₇ cycloalkyl, or C₁₋₃ haloalkyl.

4. The antibody-drug conjugate of claim 1, wherein the compound of Formula 1 above is represented by any one of the following Formulas 2 to 11:

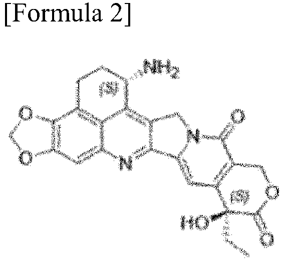

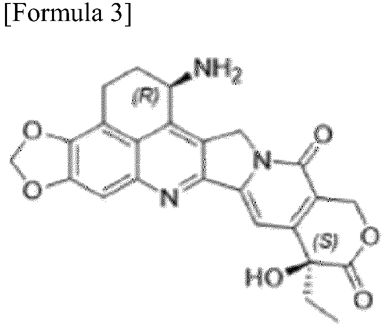

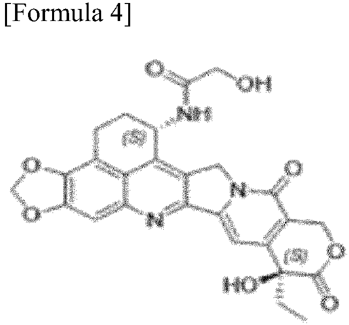

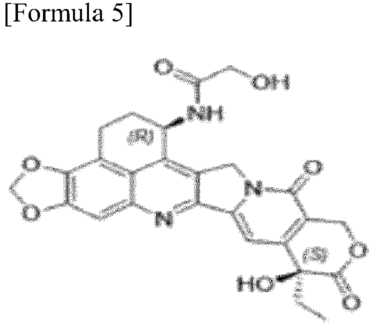

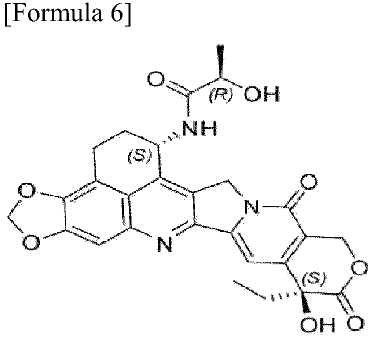

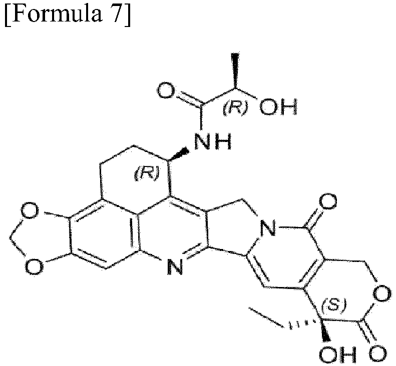

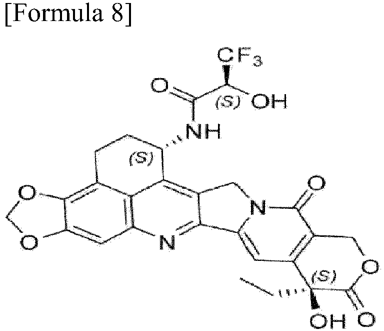

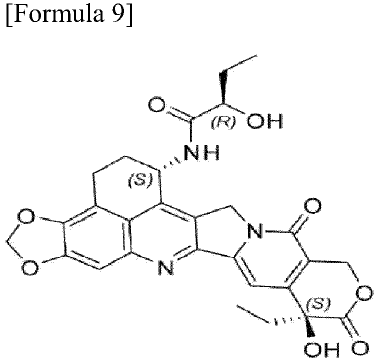

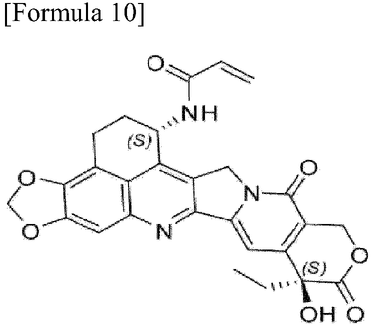

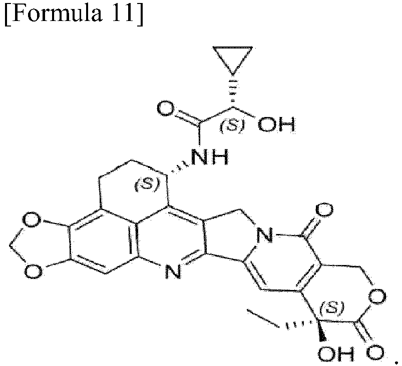

5. The antibody-drug conjugate of claim 1, wherein the linker is represented by Formula 12 below: 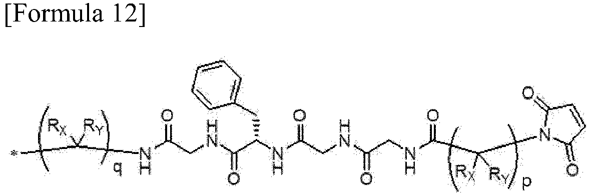 wherein:
R_{X} and R_{Y} are each independently hydrogen or C₁₋₆ alkyl;
p and q are each independently an integer of 1 to 6; and
* is the part that binds to a payload.

6. The antibody-drug conjugate of claim 1, wherein the anti-c-Met antibody is selected from the group consisting of telisotuzumab, emibetuzumab (LY2875358), onartuzumab, ARGX-111, HLX55, and M01.

7. The antibody-drug conjugate of claim 1, wherein the anti-c-Met antibody is an antibody having i) a heavy chain comprising CDR-H1, CDR-H2, and CDR-H3 having the amino acid sequences of SEQ ID NOs: 1, 2 and 3, respectively, and ii) a light chain comprising CDR-L1, CDR-L2, and CDR-L3 having the amino acid sequences of SEQ ID NOs: 4, 5, and 6, respectively; or an antigen-binding fragment thereof.

8. The antibody-drug conjugate of claim 7, wherein the anti-c-Met antibody is an antibody comprising i) a heavy chain variable region having the amino acid sequence of SEQ ID NO: 7, and ii) a light chain variable region having the amino acid sequence of SEQ ID NO: 8; or an antigen-binding fragment thereof.

9. The antibody-drug conjugate of claim 7, wherein the anti-c-Met antibody is an antibody comprising i) a heavy chain having the amino acid sequence of SEQ ID NO: 9, and ii) a light chain having the amino acid sequence of SEQ ID NO: 10; or an antigen-binding fragment thereof.

10. A pharmaceutical composition for treating or preventing cancer, comprising the antibody-drug conjugate according to any one of claims 1 to 9 as an active ingredient.

11. The pharmaceutical composition of claim 10, wherein the cancer is selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungoides, acute myeloid leukemia, acute lymphoblastic leukemia, basal cell carcinoma, ovarian epithelial cancer, ovarian germ cell cancer, male breast cancer, brain cancer, pituitary adenoma, multiple myeloma, gallbladder cancer, biliary tract cancer, colorectal cancer, chronic myeloid leukemia, chronic lymphocytic leukemia, retinoblastoma, choroidal melanoma, ampulla of Vater cancer, bladder cancer, peritoneal cancer, parathyroid cancer, adrenal cancer, paranasal sinus and nasal cavity cancer, non-small cell lung cancer, tongue cancer, astrocytoma, small cell lung cancer, pediatric brain cancer, pediatric lymphoma, pediatric leukemia, small intestine cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, uretheral cancer, cancer of unknown primary site, gastric lymphoma, gastric cancer, gastric carcinoid, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, rectal carcinoid, vaginal cancer, spinal cancer, acoustic neuroma, pancreatic cancer, salivary gland cancer, Kaposi's sarcoma, Paget's disease, tonsil cancer, squamous cell carcinoma, pulmonary adenocarcinoma, lung cancer, pulmonary squamous cell carcinoma, skin cancer, anal cancer, rhabdomyosarcoma, laryngeal cancer, pleural cancer, blood cancer, and thymic cancer.

12. The pharmaceutical composition of claim 10, wherein the cancer is selected from the group consisting of liver cancer, colorectal cancer, lung cancer, non-small cell lung cancer, gastric cancer, kidney cancer, and pancreatic cancer.

13. A method for treating or preventing cancer, comprising administering to a subject an antibody-drug conjugate according to any one of claims 1 to 9.

14. Use of the antibody-drug conjugate according to any one of claims 1 to 9 for treating or preventing cancer.

15. Use of the antibody-drug conjugate according to any one of claims 1 to 9 for preparing a pharmaceutical composition for treating or preventing cancer.
